(19) Europäisches Patentamt / European Patent Office / Office européen des brevets

(11) **EP 3 844 765 B1**

(12) **EUROPEAN PATENT SPECIFICATION**

(45) Date of publication and mention
of the grant of the patent:
**07.08.2024   Bulletin 2024/32**

(21) Application number: **19758734.8**

(22) Date of filing: **27.08.2019**

(51) International Patent Classification (IPC):
*G16H 20/17* (2018.01)     *G16H 40/63* (2018.01)
*G16H 50/20* (2018.01)     *G16H 50/30* (2018.01)
*G06F 9/455* (2018.01)

(52) Cooperative Patent Classification (CPC):
**G16H 20/17; G16H 40/63; G16H 50/20;**
**G16H 50/30;** G06F 9/453; G06F 9/45533

(86) International application number:
**PCT/EP2019/072872**

(87) International publication number:
**WO 2020/043734 (05.03.2020 Gazette 2020/10)**

(54) **SYSTEMS AND METHODS FOR PROVIDING CONTAINER BASED MEDICATION DOSE GUIDANCE TO TREAT DIABETES**

SYSTEME UND VERFAHREN ZUR BEREITSTELLUNG EINER CONTAINERBASIERTEN MEDIKAMENTENDOSISANLEITUNG ZUR BEHANDLUNG VON DIABETES

SYSTÈMES ET PROCÉDÉS POUR FOURNIR UN GUIDAGE DE DOSAGE DE MÉDICAMENTS PAR CONTENEUR POUR TRAITER LE DIABÈTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2018   US 201862723744 P**
**26.11.2018   EP 18208299**

(43) Date of publication of application:
**07.07.2021   Bulletin 2021/27**

(73) Proprietor: **Novo Nordisk A/S**
**2880 Bagsværd (DK)**

(72) Inventors:
• **MICHELICH, Alan, John**
**Seattle, Washington 98109 (US)**
• **MILLER, Thomas, Dedenroth**
**2880 Bagsværd (DK)**

(56) References cited:
WO-A1-2018/064568     US-A1- 2014 032 194
US-A1- 2017 091 419     US-A1- 2017 302 589
US-A1- 2018 137 254     US-A1- 2018 165 129

**Description**

The present disclosure generally relates to systems and methods for assisting patients and health care practitioners in managing medication dosage, in which dosages of prescribed medications are calculated based on information specific to each patient.

**BACKGROUND OF THE INVENTION**

Diabetes mellitus is characterized by impaired insulin secretion and variable degrees of peripheral insulin resistance leading to hyperglycemia. Type 2 diabetes mellitus is characterized by progressive disruption of normal physiologic insulin secretion. In healthy individuals, basal insulin secretion by pancreatic $\beta$ cells occurs continuously to maintain steady glucose levels for extended periods between meals. Also in healthy individuals, there is prandial secretion in which insulin is rapidly released in an initial first-phase spike in response to a meal, followed by prolonged insulin secretion that returns to basal levels after 2-3 hours. Years of poorly controlled hyperglycemia can lead to multiple health complications. Diabetes mellitus is one of the major causes of premature morbidity and mortality throughout the world.

Effective control of blood/plasma glucose can prevent or delay many of these complications but may not reverse them once established. Hence, achieving good glycemic control in efforts to prevent diabetes complications is the primary goal in the treatment of type 1 and type 2 diabetes. In particular, frequent changes in insulin dosage titration are key to helping stabilize blood glucose levels in patients (Bergenstal et al., "Can a Tool that Automates Insulin Titration be a Key to Diabetes Management?" Diabetes Tech. and Thera. 2012, 14(8) 675-682). Smart titrators with adjustable step size and physiological parameter estimation and pre-defined fasting blood glucose target values have been developed to administer insulin medicament treatment regimens. Optimal initiation and titration methods for the long-acting basal insulins are still being determined. However, evidence suggests that many patients often do not receive insulin doses titrated sufficiently to achieve target levels of glucose control (remaining on suboptimal doses and failing to reach treatment targets) (Holman et al., "10-year follow-up of intensive glucose control in type 2 diabetes," N. Engl. J. Med. 2008, 359: 1577-1589).

One of the major problems with insulin regimens is the lack of patient autonomy and empowerment. Patients often must visit clinics to have new titrations calculated. When a clinic has to titrate the insulin dosages for the patient, there is a natural limitation on the possible frequency of changing the titration dose. Self-titration regimens facilitate empowerment of patients, allowing them to become more involved in their treatment, which can result in improved glycemic control (Khunti et al., "Self-titration of insulin in the management of people with type 2 diabetes: a practical solution to improve management in primary care," Diabetes, Obes., and Metabol. 2012, 15(8) 690-700). Patients who take an active role in the management of their diabetes and titration of their insulin may feel more empowered to take charge of their self-care and have a stronger belief that their actions can influence their disease, thus leading to better treatment outcomes (Norris et al., "Self-management education for adults with type 2 diabetes: a meta-analysis on the effect of glycemic control. Diabetes Care," 2002, 25:1159-71, Kulzer et al., "Effects of self-management training in type 2 diabetes: a randomized, prospective trial," Diabet. Med. 2007, 24:415-23, Anderson et al., "Patient empowerment: results of a randomized controlled trial," Diabetes Care. 1995, 18:943-9). Further, when patients have control of their own titration, the frequency of titrations increases, which increases the likelihood that patients will achieve desired blood glucose levels.

Much work has been invested in the field to develop tools to enable autonomous, patient-driven titration. And significant advancements have been made in developing titration algorithms (Edelman et al., "AUTONOMY: The First Randomized Trial Comparing Two Patient-Driven Approaches to Initiate and Titrate Prandial Insulin Lispro in Type 2 Diabetes," 2014, 37(8): 2132-2140). However, many titration algorithms continue to be used primarily by health care providers during clinical visits, instead of patients, thus limiting the number of titrations each patient receives (Arnolds et al., "Common Standards of Basal Insulin Titration in T2DM," J. Diabetes Sci. Technol. 2013, 7(3): 771-788). What is needed is a fully autonomous medication dose guidance system that is accessible to patients whenever they choose or on a health care provider recommended schedule, not only when they have an appointment in a health clinic.

The structure of autonomous medication dose guidance systems poses several difficulties, including computational complexity and cost to provide. An autonomous medication dose guidance system, available on demand to patients, can be built using two parts: a client-side mobile application, and a server-side backend. The backend server can be partitioned into a database, and an engine, with the engine being where most of the system's computationally intensive functions reside. Server-side backend systems are often deployed within a cloud environment to take advantage of the scalability of cloud computing. The cloud, offered by companies such as Amazon (AWS), IBM (Bluemix), Microsoft (Azure), is most commonly accessible as an "infrastructure as a service" (IaaS). As more patients use an autonomous dose guidance system, the cloud-based engine, where most of the computing resources are required, would be allocated additional resources as needed to avoid a system time out. Cloud computing can be prohibitively expensive and time-consuming depending on the computational complexity or the volume of queries (e.g. the difficulty of calculating a dose guidance recommendation or the number of patients requesting dose guidance) (Berriman et al., "The Application of Cloud Computing to Scientific Workflows: A Study of Cost and Performance," Phil. Trans. R. Soc. A 2013, 371: 20120066).

The status quo would be to develop the engine using monolithic architecture and deploy it on a cloud-based hypervisor system (Lim et al., "Automated Control in Cloud Computing: Challenges and Opportunities," ACD'09, Barcelona, Spain. 2009). These monolithic applications are deployed in a hypervisor system that runs virtual machines (VM). Any time that demand increases (*e.g.* more patients start requesting dose guidance), the hypervisor must create additional VMs to meet the demand. This comes with substantial computing overhead because the underlying Operating System (OS) in a VM takes time, memory, and disk space needed for the VM storage. If one engine sub-function requires more computing power than other sub-functions, it can act as a resource bottleneck for the entire system and will force an extraneous and avoidable devotion of additional VMs to keep up with demand. A solution that does not require the level of overhead in a VM is needed to make cloud-based systems useful for calculating patient drug titrations.

US 2017/0091419 discloses a monitoring and treatment dosage system implemented in a cloud computing environment based on virtual machines. US 2018/165129, WO 2018/064568 and US 2017/302589 disclose general properties of a container based cloud computing system.

Thus, the problem to be solved is to maximize the efficiency of the engine and its use of computing resources when giving medication dose guidance in an IaaS cloud model. With an efficient and cost-effective model for calculating insulin titration doses, more patients will have access to on-demand autonomous medication dose titrations. The information disclosed in this background section is only for enhancement of understanding of the general background of the invention and should not be taken as an acknowledgment or any form of suggestion that this information forms the prior art already known to a person skilled in the art.

Given the above background, an object of the invention is to provide devices, systems and methods for providing improved systems and methods for distributed resource management of computationally intensive tasks within a cloud computing environment in relation to medication dosing guidance.

## DISCLOSURE OF THE INVENTION

In the disclosure of the present invention, embodiments and aspects will be described which will address one or more of the above objects or which will address objects apparent from the below disclosure as well as from the description of exemplary embodiments.

The invention is defined by the appended claims.

In a first aspect of the invention a computing system for providing medication dosing guidance recommendations to a plurality of subjects using a multi-function dose guidance algorithm comprising a plurality of functions is provided, wherein each respective function in the plurality of functions is associated with a corresponding container class, and wherein the computing system comprises one or more processors and a memory. The memory comprises instructions that, when executed by the one or more processors, perform a method responsive to receiving a medication dose guidance request from a subject in the plurality of subjects. The method comprises the steps of: (i) identifying a plurality of function requests required to satisfy the medication dose guidance request, wherein a function request in the plurality of function requests is executable by a corresponding function in the plurality of functions, (ii) using an encoder module to embed, for each respective function request in the plurality of function requests, the respective function request and associated input data into a container of the container class associated with the function corresponding to the respective function request, (iii) collecting, for each respective function request in the plurality of function requests, a function result from the container that executed the respective function request, thereby obtaining a plurality of function results, and (iv) providing a medication dose guidance recommendation using the plurality of function results.

By this arrangement the backend engine and its functions can be broken up into containers within a distributed microservice architecture. Containers (operating-system-level virtualization), are a lightweight approach to virtualization that only provides the bare minimum that an application requires to run and function as intended. By containerizing each sub function of the engine, it enables it to run reliably in different environments by abstracting away the operating system and the physical infrastructure

In an exemplary embodiment the plurality of functions comprises a first function and a second function, the first function being executable by each container of a first container class, the second function being executable by each container of a second container class, and a hardware resource requirement of the first function being different from a hardware resource requirement of the second function.

The plurality of functions may comprise a first function and a second function, wherein the first function is executable by each container of a first container class, the second function is executable by each container of a second container class, each container of the first container class is hosted by server in a first set of servers, and each container of the second container class is hosted by a server in second set of servers other than the first set of servers.

According to the invention, a composite resource requirement for the containers of a given container class is evaluated on a recurring basis, and when the composite resource requirement for the containers of the given container class satisfies a resource allocation threshold associated with the given container class, one or more additional containers of the given container class is added and permitted to accept function requests from the encoder module that match the given container class.

Correspondingly, when the composite resource requirement for the containers of the given container class satisfies a second resource allocation threshold associated with the given container class, one or more containers of the given container class is removed and no longer permitted to accept function requests from the encoder module.

In an exemplary embodiment a given container of a container class associated with the function corresponding to a given function request evaluates whether the respective function request contains input data required for the function to provide a function result, and when the function request fails to contain the input data required, no function result is provided by the given container of the container class associated with the function.

The computing system may be operated to concurrently execute a medication dose guidance request for each subject in the plurality of subjects. The plurality of subjects may comprise at least 10,000 subjects.

The plurality of functions may comprise one or more of calculating a medication dose guidance recommendation of a subject, reconstructing an insulin injection history of a subject, calculating a titration glucose level of a subject, reconstructing a blood glucose history of a subject, and getting dose guidance parameters of a subject.

The medication dose guidance request is for a dose of insulin medicament in order to achieve a predetermined blood glucose target range of a subject to treat a diabetes mellitus condition.

In an exemplary embodiment the method comprises the further step of performing a quality check of the dose guidance request, wherein the quality check fails and results in no medication dose guidance recommendation being provided when (a) a dose guidance request from a subject comprises a record of a hypoglycemic event, or (b) a dose guidance request from a subject comprises a record indicating that the subject has failed to take a previous recommended medication dose.

The encoder module (ii) may further comprise evaluating each respective function for a resource requirement.

At least two functions for a subject may be concurrently executed, and/or at least two functions for a subject may be executed sequentially.

In an exemplary embodiment the input required by the function request comprises, for a given subject, at least an upper limit target glucose range of the subject, a blood glucose history of the subject, a basal injection history of the subject, and a most recent medication dose guidance recommendation, and optionally one or more of a body weight of the subject, a lower limit target glucose range of the subject, an overbasalisation limit of the subject, and a starting insulin basal dose of the subject.

In a second aspect the present disclosure addresses the above-identified need in the art by providing systems and methods for medication dosage guidance, particularly for use in between visits to a health care practitioner. In the present disclosure, a computing system for providing medication dosing guidance recommendations to a plurality of subjects uses a multi-function dose guidance algorithm comprising a plurality of functions. Each respective function in the plurality of functions is associated with a container class in a plurality container classes. The computing system comprises one or more processors and a memory. The memory comprises instructions that, when executed by the one or more processors, perform a method responsive to receiving a medication dose guidance request from a subject in the plurality of subjects. The method comprises: (i) identifying a plurality of function requests required to satisfy the medication dose guidance request, where a function request in the plurality of function requests is executable by a corresponding function in the plurality of functions, (ii) using an encoder module to embed, for each respective function request in the plurality of function requests, the respective function request into a container in a container class associated with the function corresponding to the respective function request, (iii) collecting, for each respective function request in the plurality of function requests, a function result from the container in the container class that executed the respective function request, thereby obtaining a plurality of function results, and (iv) providing a medication dose guidance recommendation using the plurality of function results.

In some embodiments, the plurality of functions comprises a first function and a second function. The first function is executable by each instance of a container of a first container class in the plurality of container classes. The second function is executable by each instance of a container of a second container class in the plurality of container classes. A hardware resource requirement of the first function is different than a hardware resource requirement of the second function.

In one aspect of the present disclosure, the system concurrently executes a medication dose guidance request for each subject in the plurality of subjects, e.g. at least 10.000 subjects. The first function and the second function for a subject may be concurrently or sequentially executed.

According to the invention, a composite resource requirement of a first set of instances of containers of a first container class is evaluated on a recurring basis. When the composite resource requirement of the first set of instances of containers of the first container class satisfies a first resource allocation threshold associated with the first set of containers, one or more additional instances of containers of the container class is added to the first set of containers and permitted to accept function requests from the encoder module that match the type of the first container class.

When the composite resource requirement of the first set of instances of the first container class satisfies a second resource allocation threshold associated with the first set of containers, one or more containers in the first set of containers is removed from the first set of containers and no longer permitted to accept function requests from the encoder module.

In some embodiments, the plurality of functions comprises a first function and a second function. The first function is executable by each instance of a container of a first container class in the plurality of container classes. The second function is executable by each instance of a container of a second container class in the plurality of container classes. Each instance of a container of the first container class is hosted by server in a first set of servers. Each instance of a container of the second container class is hosted by a server in second set of servers.

In an exemplary embodiment of the computing system the plurality of functions comprises a first function and a second function, the first function being executable by each instance of a container of a first container class in the plurality of container classes, and the second function being executable by each instance of a container of a second container class in the plurality of container classes. Each instance of a container of the first container class is hosted by server in a first set of servers, and each instance of a container of the second container class is hosted by a server in second set of servers other than the first set of servers.

The first function may comprise one of (i) calculating a medication dose guidance recommendation of a subject, (ii) reconstructing an insulin injection history of a subject, (iii) calculating a titration glucose level of a subject, (iv) reconstructing a blood glucose history of a subject, or (v) getting dose guidance parameters of a subject.

The reconstructing a blood glucose history of a subject may further comprise: calculating a reconstructed blood glucose history of the subject when the blood glucose history time course contains a gap, and wherein the reconstructed blood glucose history is calculated based on the blood glucose history of the subject each calendar day.

In a further exemplary embodiment of the computing system the container in the container class associated with the function corresponding to the respective function request evaluates whether the respective function request contains input required for the function to provide a function result, wherein, when the function request fails to contain the input required, no function result is provided by the container in the container class associated with the function.

The input required by the function request comprises, for a given subject, at least (i) a body weight of the subject, (ii) an upper limit target glucose range of the subject, (iii) a lower limit target glucose range of the subject, (iv) an over-basalisation limit of the subject, (v) a blood glucose history of the subject, (vi) a most recent medication dose guidance recommendation and/or a starting insulin basal dose of the subject, and (vii) a basal injection history of the subject.

The upper limit target glucose range used to determine the medication dose guidance recommendation may be within 80-180mg/dL, 90-180 mg/dL, 100-180mg/dL, 90-200mg/dL, 90-250mg/dL or 90-300mg/dL.

The lower limit target glucose range used to determine the medication dose guidance recommendation may be within 50-70mg/dL, 70-90 mg/dL, 70-100mg/dL, 60-100mg/dL, or 60-90mg/dL.

According to the invention, the medication dose guidance request is for a dose of insulin medicament, wherein the dose of an insulin medicament is to achieve a predetermined blood glucose target range of a subject to treat a diabetes mellitus condition. The condition may be type 2 diabetes mellitus and the insulin medicament may be LysB29(Nc-hexadecandioyl-γ-Glu) des(B30) human insulin (insulin degludec, Tresiba®).

The predetermined blood glucose target range is 50-180mg/dL, 60-180mg/dL, 70-180mg/dL, 80-180mg/dL, 50-200mg/dL, 60-200mg/dL, 70-200mg/dL, 80-200mg/dL, 50-250mg/dL, 60-250mg/dL, 70-250mg/dL, or 80-250mg/dL.

In a yet further exemplary embodiment of the computing system the method further comprises performing a quality check of the dose guidance request, wherein the quality check fails, and wherein (a) the subject records a hypoglycemic event or (b) the subject has failed to take a previous recommended medication dose, a medication dose guidance recommendation is provided.

In a further exemplary embodiment of the computing system the encoder module (ii) further comprises evaluating each respective function for a resource requirement.

## BRIEF DESCRIPTION OF THE DRAWINGS

In the following embodiments of the invention will be described with reference to the drawings, wherein

figs. 1A and 1B show a flowchart of processes and features for a first embodiment of a system providing a dose guidance recommendation,
figs. 2A and 2B illustrates how a TGL value is determined based on CGM data,
fig. 3 illustrates an example system of container organization for autonomously providing a medication dose guidance recommendation, in accordance with an embodiment of the present disclosure,
figs. 4A and 4B collectively illustrate exemplary system topologies that includes a data collection device for collecting patient data (e.g., data from one or more glucose sensors that measure glucose data from the subject), a medication dose guidance request system, and one or more container engines that each include an orchestrator and one or more containers, where the above-identified components are interconnected, optionally through a communications network, in accordance with an embodiment of the present disclosure,

fig. 5 illustrates a computer system for autonomously providing a medication dose guidance recommendation, in accordance with an embodiment of the present disclosure.

fig. 6 provides a flow chart of processes and features of a device for autonomously providing a medication dose guidance recommendation, in accordance with various embodiments of the present disclosure,

fig. 7 illustrates a medication dose guidance request for a subject, in accordance with an embodiment of the present disclosure,

fig. 8 illustrates an example integrated system of connected insulin pen(s), continuous glucose monitor(s), memory and a processor for autonomously providing medication dose guidance recommendations, in accordance with an embodiment of the present disclosure, and

fig. 9 illustrates an example system of container organization for autonomously providing a medication dose guidance recommendation, in accordance with a further embodiment of the present disclosure.

In the figures like structures are mainly identified by like reference numerals.

## DESCRIPTION OF EXEMPLARY EMBODIMENTS

Diabetes is a growing world health epidemic. Although diabetes can be effectively managed with established titration treatment regimens and pharmaceuticals, the access to up-to-date titration recommendations remains limited. The present disclosure provides, a patient-focused titration algorithm that would facilitate automated medication dosage adjustments and self-titration, for both insulin and other drugs, thus enhancing patient empowerment as well as substantially reducing treatment costs by reducing the frequency of required physician consultations for dose adjustments without reducing therapeutic outcomes. The present disclosure describes a system that provides medication dose guidance recommendations either upon request from patients or autonomously. One aspect of the preset disclosure is that the integrated system for providing medication dose recommendations is based in a cloud ecosystem. Before describing embodiments of the present invention *per se*, a specific example of how a dose guidance request (DGR) is received and processed when stored instructions are executed will be described. The number of functions, the order of the functions and the specific functionality, e.g. rules, embedded in each given function are all exemplary. In the exemplary embodiment the described rules correspond to the titration label recommendations for Tresiba® from Novo Nordisk A/S.

The system setup that will be described in the following is designed to be implemented as a backend engine adapted to be used as part of a cloud-based large-scale diabetes management system. Such a cloud-based system would allow the engine to always be up-to-date (in contrast to app-based systems running entirely on e.g. the patient's smartphone), would allow advanced methods such as machine learning and artificial intelligence to be implemented, and would allow data to be used in combination with other services in a greater "digital health" set-up. As such a cloud-based system ideally would handle a large amount of patient requests for dose recommendations, it is important that the system is set up to provide the intended service as quickly as possible and with an effective use of the available resources.

Overall a diabetes management system is provided that helps people with type 2 diabetes and their health care providers (HCPs) titrate basal insulin in response to blood glucose values and the amount of insulin recorded in previously logged doses. The complete system comprises the back-end engine ("the engine") which is the main aspect of the present invention used in combination with interacting systems in the form of a client and an operating system.

The client from the engine's perspective is the software component that requests dose guidance. The client gathers the necessary data (e.g. CGM data, injection data, patient parameters) and requests dose guidance from the engine. The client then receives the response from the engine. In a typical set-up the client may be a dedicated data hub for acting as an interface between the patient's medical devices and the cloud or it may be an app running on a patient smartphone. The cloud operating system provides the engine with the minimum resources necessary to function (e.g. memory, processing). The cloud operating system acts as the environment in which the engine is installed and allows for the deployment thereof.

In the following embodiments of a system for providing a long-acting or ultra-long-acting insulin adjustment day dose recommendation (ADDR) for a subject to treat diabetes mellitus will be described, the system being suitable for implementation as a cloud-based large-scale diabetes management system.

More specifically, a system for providing a long-acting or ultra-long-acting insulin adjustment day dose recommendation (ADDR) for a subject to treat diabetes mellitus is provided. The system comprises one or more processors and a memory, the memory comprising instructions that, when executed by the one or more processors, perform a method responsive to receiving a dose guidance request (DGR).

The instructions provide that a first data structure is obtained, comprising a glucose upper target range level (UTR) of the subject, and a glucose lower target range level (LTR) of the subject, and that a second data structure is obtained, comprising a current dose guidance baseline (DGB), and a corresponding DGB timestamp representing when the DGB was made. The current DGB may correspond to either a most recent adjusted day dose recommendation, or a starting basal dose, i.e. the basal dose typically set by the patient's doctor. As described above, these data structures represent the starting point for the titration regiment (the starting dose) respectively the target range for the patient's BG level.

In order to be able to calculate an updated ADDR the instructions provide that a first and second data set is obtained. The first data set comprises a plurality of glucose measurements of the subject taken over a time course and thereby establishing a blood glucose history (BGH), each respective glucose measurement comprising a blood glucose (BG) value, and a corresponding glucose timestamp (BGT) representing when in the time course the respective glucose measurement was made. The BG values may be in the form of stand-alone BG values, typically a daily measurement taken in the morning and representing a fasting BG. In this case each BG value represents a what can be termed a daily titration glucose level value (TGL) upon which the calculation of an updated ADDR can be based. Alternatively, the first data set may comprise a large number of BG values compiled over each day by use of a skin-mounted continuous glucose monitoring (CGM) device, e.g. providing a BG value every 5 minutes. Based on the CGM data a daily TGL can be determined using an appropriate algorithm as will be described in greater detail below.

The second data set comprises the basal insulin injection history (IH) of the subject, the history comprising a plurality of injections during all or a portion of the time course with each respective injection being characterized by an injection amount (IA), and an injection timestamp (IT) representing when in the time course the respective injection occurred. To assure that the injection history is up-to-date a third data structure comprising an injection history refresh timestamp may be obtained. The injection data may have been captured manually by the patient or they may have been captured using a drug delivery device with provided with dose logging circuitry. In the latter case it should be noted that expelled dose amounts and not necessarily injected dose amounts are logged. Indeed, this inaccuracy potentially also applies to a manually derived dose log. The issue of non-injected priming/air shots will be discussed below.

The instructions further provide that the data structures and data sets are evaluated to thereby determine whether all the data types necessary in a dose guidance request are present and within a specified range. This includes BG data, injection data, target glucose range upper and lower limits and most recent adjustment day dose recommendation or starting basal dose. This is to ensure that if something is missing, or clearly wrong or insufficient, resources are not wasted trying to process the request. In the event that a request lacks a data type or has a parameter that is an order of magnitude higher than what is considered normal, the engine will return a corresponding error message. If all data types are present and within range (quality and or quantity are not considered here), the check passes the data along for further processing.

Before calculating an ADDR a number of data checks may be performed, e.g. performing a refresh check to check whether the dose guidance request was received within a given time limit from the injection history refresh timestamp, as well as an update check to check whether the dose guidance request was received within a given time limit from the DGB timestamp.

Further, one or more dose event checks may be performed to determine whether the number, timing and size of the individual injections in the injection history (IH) conform to a set of pre-determined compliance requirements.

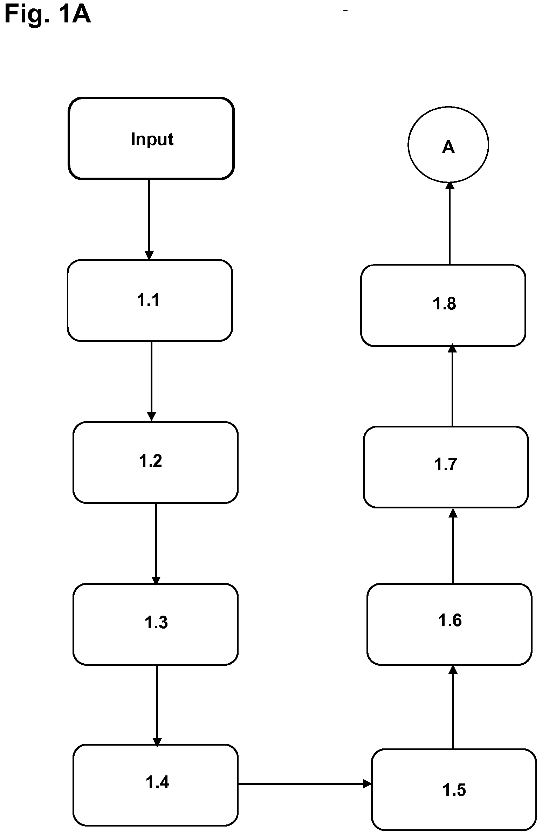

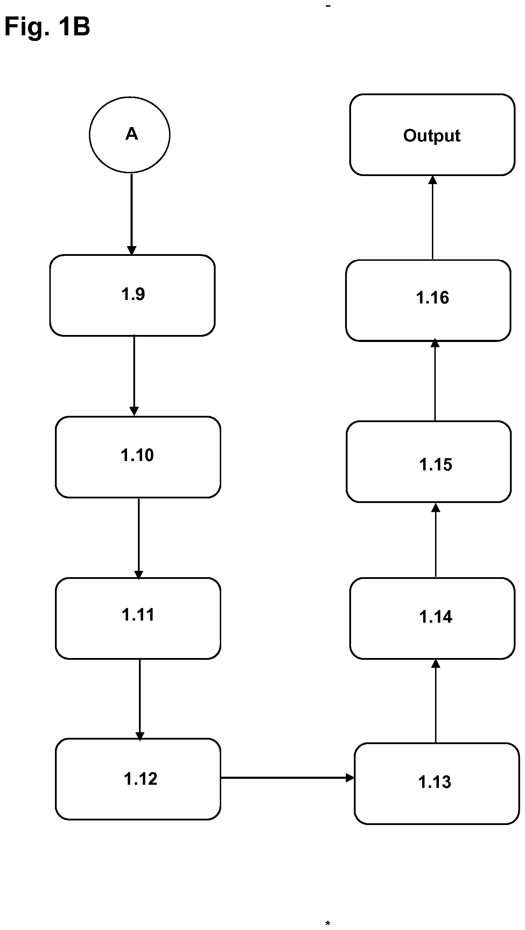

Having described the basic components of an exemplary embodiment of the invention, a specific embodiment will be described with reference to fig. 1 in which a flow-chart illustrates how a dose guidance request (DGR) is received and processed when the stored instructions are executed. The number of functions, the order of the functions and the specific functionality, e.g. rules, embedded in each given function are all exemplary. In the exemplary embodiment the described rules correspond to the titration label recommendations for Tresiba® from Novo Nordisk A/S.

## 1.1 Valid Request Check

This function is described above and checks that all the data types necessary in a dose guidance request (i.e. client input data) are present and within a specified range. In the event that a request lacks the appropriate data the function will returns an error message. Otherwise the check passes the data along.

For a given set-up additional input data may be required for a request to pass the check, e.g. a "hypo history" including any reported hypoglycemic events with a timestamp, a max basal limit for a given period of time, e.g. per day or per week, the time of request (TOR), a unique identifier of an individual dose guidance request, and a unique identifier of a client.

## 1.2 Last Injection Data Refresh Check

This function checks that the injection history data has been refreshed within a predetermined time limit, e.g. the last 30 seconds, this is to ensure that it has the most up to date record of the injection history data.

### 1.3 Injection History Check

This function checks whether there was already a dose event within the past 8 hours. If so, recommending another injection would violate the Tresiba® labelling (i.e. take an injection once daily and at least 8 hours apart) and put the user at risk for a hypoglycemic event. If there are no dose events within the previous 8 hours, the check will pass the data along to the next function.

### 1.4 Make-up injection check

The Tresiba® labelling states that the user should take an injection once daily and at least 8 hours apart. But in the event that the patient forgets to take their daily dose, they can take the forgotten dose the next day in addition to their regularly scheduled dose (spaced out by at least 8 hours). Correspondingly, this function first checks to see if there's already been a dose event within the calendar day. If so, it then checks to see if the previous calendar day is without a dose event, and if this is true, it will pass the check. If there is a dose event recorded from the previous calendar day, the check will return an error message and not give a dose recommendation.

### 1.5 Recorded hypoglycemic event check

This function checks whether the data structure received with the request comprises an indicator for one or more recorded hypoglycemic events. If one or more hypoglycemic events have been recorded within a preset time limit no new ADDR will be calculated but the current DGB will be adjusted down with 2 III of insulin. Hypoglycemic events may be recorded manually by the patient and/or may be registered automatically when a CGM is used for BG data capture. In the latter case the patient may be requested to confirm the event.

### 1.6 BG data quality check

In case BG data is supplied based on individual BG measurements, e.g. fasting BG, the function will check that a valid BG value is recorded for a specified number of days out of a specified number of recent days, e.g. at least 3 valid BG values for the last 4 days. In case BG data is supplied based on CGM measurements a CGM data quality check may be performed to ensure that there are no unacceptable gaps in the data that may lead to a wrong titration glucose level determination. Exemplary criterions for what an unacceptable level of CGM data quality is are described below. In the event that the data quality is not able to meet the minimum threshold, this function will return an error message and the dose guidance request will not proceed. Alternatively, it may be possible to fill in gaps within the CGM data. This is to increase the probability that despite gaps in the data (e.g. a new sensor is warming up) a dose guidance request will pass the next CGM data quality check. Examples of suitable mathematical approaches will be described in greater detail below.

### 1.7 Dose event identification

In a pen device with integrated dose logging functionality such as NovoPen® 6, according to its instructions for use, the patient should prime the device until they see insulin squirt out. These priming "injections" (or "air shots") are not differentiated from actual body injections by the pen and would be seen from the engine's perspective as individual injections taken into the body. As the number of actual injections taken by the patient is one of the parameters that may be checked by the dose guidance system to determine whether the patient is in compliance with the titration regimen, it is relevant to be able to filter out such priming "injections". This filtering could take place in the dose logging circuitry (whether integrated as in NovoPen® 6 or provided as an add-on logging device to be mounted on a pen device), in the patient's smartphone app which will typically be the device adapted to receive and collect injection data and BG data before they are transmitted as part of a request for an ADDR, or in the cloud engine. The filtering will typically be based on dose size (priming doses are generally (much) smaller than injection doses, and time between injections. A number of algorithms performing this analysis are known, e.g. as disclosed in US 2019/0035500. The filtering may be implemented at more than one level, as a subsequent filtering would just filter out nothing. The same considerations apply to "split doses", i.e. a (typically) large dose which by the patient is split into two separate injections, which should be combined to a "dose event", see below, in order not to count as two individual doses which would jeopardize a titration regimen based on the number of dose events.

### 1.8 Dose event check

This function is checking to see if the patient has been sufficiently adherent in his/her injection routine. It looks for at least 3 dose events since the most recent ADDR, and that they have occurred within today and up to a previous 4 calendar day window. This extra calendar day that is being checked allows for a day that is without a dose (e.g. he/she forgot) and subsequently takes it the next day - in addition to the normally scheduled dose. If there are less than 3 dose events in this time frame (because the patient forgot multiple days) than this check will fail, and it will lead to a re-recommendation of the most recent ADDR made. Additionally, it will ensure only a single ADDR is dispensed and only re-recommendations follow in a scenario where repeated requests come in for an ADDR without any injections having since taken place.

### 1.9 DGB check

This function checks to see when the most recent ADDR was made - or when the starting basal dose (SBD) value was set. If the most recent ADDR was made within the current and previous 7 calendar days, then the check passes. If the most recent ADDR is older than this, than a new SBD amount must be inputted, and the dose guidance request process must be re-started from this new point. The new SBD would then be considered the current DGB.

### 1.10 Adherence check

This function checks the injection amounts of the three most recent dose events in the dataset to ensure that they are each at least equal to or greater than the amount of the most recent ADDR. If any of the dose events are less than this amount, the check will fail, and it will re-recommend the amount from the most recent ADDR. This ensures that the engine does not titrate up based on elevated glucose levels which are due to under-dosing.

### 1.11 Too many doses check

This function checks whether there are more than two dose events in the last 48 hours which would be against the recommendations of the Tresiba® label. In case of more than two dose events in the last 48 hours have been recorded then an error message may be generated.

### 1.12 Daily TGL determination

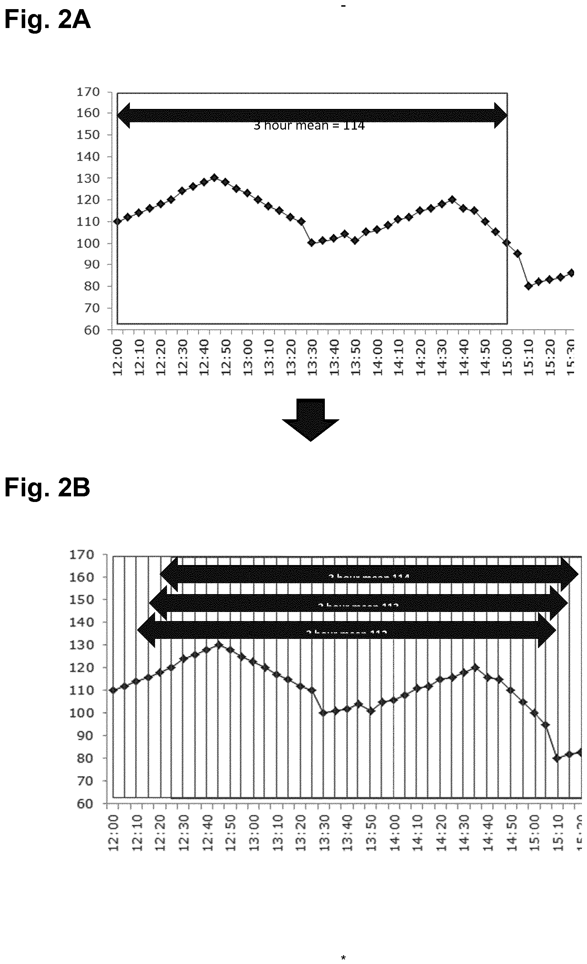

In case BG data is supplied based on individual BG measurements, e.g. fasting BG, the function will check that a valid BG value is recorded within a specified time range. In case BG data is supplied based on CGM measurements, this function looks at the CGM dataset and determines, if possible, a TGL for each day since most recent ADDR or up to a maximum of days, e.g. 4 days from the TOR, this being the dose guidance period. A "day" may be a calendar day (in which case some days would be partial), or it may be a 24 hours period calculated from e.g. the TOR, see below. It does this by applying e.g. a 3 hour "sliding window" across CGM readings one data point at the time, see figs. 2A and 2B, calculating the mean of each 3 hours interval from the current time up until the time of the last dose adjustment. In an exemplary embodiment a daily TGL is determined only for days having passed the BGH data quality test as described below. The lowest 3 hour mean for each day will be recorded as a TGL value and used for titration.

### 1.13 Daily TGL check

This check looks for any daily TGLs that are below the lower limit of the target glucose range parameter. If so, then the next ADDR is reduced with 2 IU from the most recent ADDR given. If not, the check passes onto the next function.

### 1.14 Average TGL determination

If at least a minimum number of valid daily TGL values have been determined, e.g. 2, for the dose guidance period, e.g. last 4 days, an average for the at least 2 TGL values is calculated.

### 1.15 Titration Determination

This function utilizes the overall TGL average, and if it is within the target range, the titration determination will be +0 IU from the most recent ADDR. If the TGL average is above the upper limit of the target range, then the next ADDR is increased by 2 IU from the most recent ADDR. If the TGL average is below the upper limit of the target range, then the next ADDR is lowered by 2 IU from the most recent ADDR.

**1.16 Maximum limit check**

To prevent overdosing this function checks a that a given dose maximum for a given period of time has not been exceeding, e.g. a maximum of 300 IU of Tresiba® for the last week. A patient specific value may also be included in the request data. Alternatively, the function may check whether the next ADDR would exceed the patient's "overbasalisation" limit (BW (kg) * OBL (IU/kg)). This would require that a body weight (BW) of the subject, and an overbasalisation limit (OBL) of the subject have been provided as part of the data received with the request.

**1.17 Output**

The main output from the engine as a reply to a dose guidance request is indeed the ADDR as received by the client, however, additional information may be of use to the patient, either directly related to the patient's treatment, e.g. TGL values calculated by the engine based on CGM values as well as specific error or warning messages, or validation data to improve reliability and safety. Correspondingly, the output may comprise one or more of the following types of data: User ID, transaction ID, ADDR, day TGLs, overall TGL, dose event history, hypogly-caemia history, warning and error codes.

Referring to "1.6 BG data quality check" above, in the following an exemplary criterion for an unacceptable level of CGM data quality will be described.

In general, for decision support systems that wants to provide insulin dose guidance to patients with T2DM using a CGM device, the above-described system is a back-end dose guidance engine that ingests insulin, glucose, and patient data to analyze and recommend how much basal insulin to take now, so that patients can live a more normal life.

The glucose data ingested may be from a continuous glucose monitor. Current CGMs such as a Dexcom G5 reports data every 5 minutes continuously, and it is the task of the engine to determine the Titration Glucose Level (TGL) from this continuous stream of data to ultimately make a dose recommendation. Unfortunately, today's CGMs do not have the memory to store the data, and any data that isn't received by the receiving device, e.g. a mobile app running on the patient's smartphone, is lost. This creates a challenge in safely determining a TGL while dealing with these gaps, which are likely to occur in real world use. Table 1 describes a first example of a go/no-go criterion for safely handling gaps in CGM data.

Table 1: Overview of actions based on missing CGM data

| Category | Level of data missing | Level of uncertainty | Action to be taken by the app |
|---|---|---|---|
| 1 | No data gap interval larger than 20 min. | Low level of uncertainty | Provide next insulin dose recommendation based on CGM data. |
| 2 | There are gaps longer than 20 min & there is no gap longer than 3 hours in any of the last three days before titration & no more than 60 of the normal 5-min samples are missing in any of the last three days. | Low level of uncertainty | Next insulin dose recommendation based on CGM data must take into account: The 60 min data window that contains gaps of longer than 20 min should not be used for averaging until the window passes the >20 min gap. |
| 3 | No more than one of the days (out of the last three days) has CGM gaps of longer than 3 hours or has more than 60 of the normal 5-min samples missing. | Moderate level of uncertainty | Next insulin dose recommendation based on CGM data must take into account: Exclude the day from titration, use the other two days for dose recommendation. |
| 4 | More than one of the days (out of the last three days) has CGM gaps of longer than 3 hours or has more than 60 of the normal 5-min samples missing. | High level of uncertainty | No next insulin dose must be recommended. |

In the following a further example of a BGH data quality test will be described.

A dose guidance period (DG_PERIOD) can be defined as the period of time starting from the time of request to the timestamp of the DGB, up to a maximum of e.g. -96:00 hr. The BGH data in the DG_PERIOD is the divided into the following possible increments: BGH_PERIOD_1: 0 to -24:00 hrs, BGH_PERIOD_2: -24:00 to -48:00 hrs, BGH_PERIOD_3: -48:00 to DGB time/-72:00 hrs, and BGH_PERIOD 4: -72:00 hrs to DGB time/-96:00 hrs.

To carry out the data quality test, each BGH_PERIOD is broken into non-overlapping 6-hour interval(s). Each interval is checked for the presence of at least 1 VALID_TGL being defined as a window of time where > 50% of BGH data points are present. The window may be e.g. 3 hours. If any 6-hour interval(s) is without at least 1 VALID_TGL, the entire BGH_PERIOD has failed the data quality test, i.e. each BGH_PERIOD is tested individually and will have its own pass/fail determination. Further details in respect of CGM data quality tests are disclosed in WO 2018/228932.

As mentioned above, it may be necessary to combine doses to create a "dose event". In an exemplary embodiment all injections in the IH which fall within a running DE_WINDOW of, e.g. 60 minutes, are aggregated into a DOSE_EVENT amount starting from the current time. Only Injections that are within the DG_PERIOD will be aggregated into DOSE_EVENTs. The DOSE_EVENT Timestamp is, e.g. the timestamp of the most recent injection within a DE_WINDOW. As appears, in case priming and air shot expelling events are not filtered out of the IH prior to it being submitted as part of the DGR then these non-injected dose amounts will be calculated as part of the injected dose amounts. Alternatively, the engine may be provided with its owner filter functionality, e.g. rule-based flow-check detection for connected pens as described in EP application 18198410.5.

To ensure that the disclosed engine cannot only be safe, but also effective in treating patients to target, an extra data processing step may be applied to fill in certain gaps in the CGM data where previously a TGL was unable to be determined and the system unable to determine a dose guidance recommendation. By utilizing a data gap filling method, the engine may be able to calculate basal dose guidance in a more realistic "real world" environment where data can have gaps, while the system remains safe and effective for dispensing basal insulin dose guidance.

More specifically, in the following the main idea of the method is to define lowest quasi stationary states of glucose profile and fill in data gaps with respect to them. The proposed model takes into account general trends and typical behaviour of the patient.

The model was developed based on patients' CGM data from a clinical trial with 2 weeks of CGM data at the initiation of the study with basal only therapy. Analysis of the data showed that at least 30% of patients had quite big gaps in the CGM data. These gaps were at different time of the days and nights. According to the project's risk analysis, there was a risk that these gaps could severely impact algorithm dosing decisions because the gaps could occur during fasting periods, therefore preventing the titration algorithm from being able to correctly identify the TGL and giving a wrong recommendation. The task was to perform analysis and assure that proposed approach was agnostic to the CGM noise and did not lead to hypoglycaemic events.

The CGM data gap filling processing step consists of several steps. First, it checks how much data is available in the CGM stream. The requirement is to have at least 30% of data within last X amount of days needed to identify TGL and for titration (e.g. X=3 for an exemplary algorithm implementation). Second, it needs to identify gaps in the CGM data and store them in array of gaps. It is possible to refill from 1 to 72 missing points (1 missing point = 2 intervals = 10 minutes gap). Even though 72 missing points gap looks big, the gap filling algorithm was able to reconstruct CGM data correctly.

Data analysis showed that the absolute value difference between all neighboring points on the 90th percentile and 75th percentile would be approximately 10 mg/dl and 6mg/dl for users with high glycemic variability while only 5 mg/dl and 3 mg/dl for users with low glycemic variability. The next logical step is to find all gaps between 7 to 360 minutes. The reconstruction process deals with each and every gap individually. The algorithm is as the following:

- take the last point before gap and the first one after
- find what is the typical CGM rate of change (ROC) for the particular user on the 75th percentile,
- calculate maximum of difference from the last and the first points as difference between the last (first) point value minus ROC times number of missing points (denoted x3 and x4 correspondingly),
- find an intersection between two lines and store the value of time $t_i$,

Sometimes if difference |x1 - x2| is bigger than ROC times number of missing points the intersection point will be outside of the time interval (t1, t2). It will be necessary to repeat above-mentioned part of the algorithm routine with 90% percentile ROC. If the algorithm is unable to achieve intersection point within time interval (t1, t2) with 90% percentile ROC, it will connect points with a straight line. Mathematical description for that is the following, when the time of the gap is small and the person is rising from high glycaemic index meal, the worst case scenario will be a straight line (degenerate case of a V-shape line).

Further, data analysis shows that the true fasting period values form valleys with some reasonable amount of points.

The machine learning clustering method k-Means allows to find clusters of points. Standard k-Means algorithm with random initialization of cluster centroids was used. There is a need to prepare CGM data to find clusters. The simplest way of filtering CGM data is to neglect all data bigger than 30th percentile of the stream. The advantage of this approach is that the cut-off line based on the input data is shifted, while there are still enough data points to find clusters.

Defining cluster centroids is a very powerful tool and by finding the minimum cluster centroid value it can said, that points of the cluster trending lower and closer together. The last step is to define minimum of the lowest cluster and store this value $x_c$.

After defining cluster centroids gaps can be refilled with V-shape curves, where the head of V is defined by point $[t_i, x_c]$. Since the patient's body cannot be fully reproduced, all nonlinear can be approximated with lines.

When a given BG data set has passed the BG data quality check a daily TGL determination will be performed. In the following two concepts for this purpose will be described. In contrast to the above-described exemplary embodiment a sliding window of 60 minutes was utilized.

**Approach 1:**

Detect TGL by selecting the lowest average of 60 minutes glucose levels (i.e. the lowest of 288 13 consecutive measurements of 5 minute intervals during a 24 hour period). This approach calculates a moving average (MA) of 13 consecutive measurements of 5 minute intervals,

$$MA_{60\min,i} = \frac{1}{6} \sum_{k=i-12}^{i} G_k$$

where $G$ is the glucose measurement and the subscript $i$ is the current sample. The minimum $MA_{60\min,i}$ during the 24 hrs between 00:00 and 23:59 are determined to be the TGL. This approach is hypothesised to mitigate b) people with irregular daily routines (shift workers etc.) and c) people whose daily routines deviate from normal practice (e.g. not having breakfast).

**Approach 2:**

Detect TGL by identifying the first steep curve in the time interval 04.00 to 10.00, which is assumed to represent breakfast, and then determine TGL as the average glucose levels in the time interval -90 minutes to -60 minutes. This approach calculates a backward difference as described by Dassau et al. for meal detection using CGM data:

$$\frac{dG_i}{dt} = \frac{3G_i - 4G_{i-1} + G_{i-2}}{2\Delta t}$$

where $G$ is the glucose measurement, $t$ is time, $\Delta t$ is the time difference between two sample intervals, and the subscripts $i$, $i$ - 1 and $i$ - 2 are the current and the two previous samples. dG/dt is calculated for all points in the predefined time period. Start of a meal is defined to be where the dG/dt is at maximum. TGL is then calculated as the average of the measurements in the time interval -90 minutes to -60 minutes. This approach is hypothesised to be more robust for people with a) regular and predictable lifestyles, but less robust when lifestyle irregularities are present.

The two approaches were challenged against each other by creating a number of scenarios, (e.g. for people with "typical" and predictable lifestyles, people with "atypical" daily routines, insulin sensitivity changes and CGM sensor deviations) and for each scenario generate CGM data sets based on virtual patient models.

Outcome measures were then calculated based on the continuous glucose output from the simulators. The outcome measures were used to evaluate the performance of the physiological models compared to clinical trial results, and to compare the safety and performance of the treatment decision algorithms.

Outcome measure 1: Frequency of severe hypoglycemic events
Outcome measure 2: Severities of too high or too low dose recommendations
Outcome measure 3: Time in range

When ranking the two approaches, approach 1 performed best in all simulation tests. Correspondingly, approach 1 is assessed to be a feasible means to detect and determine TGL and is assessed to be a safe approach to perform CGM based titration of basal insulin.

A practical application of approach 1 is to use the 3 24 hours periods prior to the request for an insulin dose request. I.e. the dose recommendation time = the dose time = t0. The 3 moving averages are found during 1) t-24h to t0, 2) t-48h to t-24h and 3) t-72h to t-48h. There must be a time difference between TGL periods, e.g. at least 8 hours between TGL periods. This is to avoid the scenario where the same TGL period is used in two 24 hours periods.

In the following it is assessed what data quality is required from the CGM in order to safely and reliably perform the basal insulin dose recommendation. The following assumptions apply:

- Indication: Basal insulin titration for patients with T2DM
- Titration algorithm: "Simple"2-0-2 algorithm
- Approach to detection and determination of TGL: Approach 1, see above
- The basal insulin used in the trial is insulin degludec, which has a very flat action profile with $t_{max}$ of a median of 9 hours, and $t_{\frac{1}{2}}$ of approximately 25 hours.

In principle, given these assumptions, the conservative titration algorithm combined with the conservative approach to detect and determine TGL should provide a safe automatic basal insulin titration in it-self. However, in the following the use of CGM data with missing sections is evaluated.

The proposed use of GCM data with missing sections and optimised CGM data quality is assessed using existing clinical GCM data from published clinical trial NN1218-3853 titled: "Efficacy and safety of Fiasp® compared to insulin aspart in combination with insulin glargine and metformin in adults with type 2 diabetes (Onset® 2)". The CGM data from this trial is applicable because:

- Trial includes only Type 2 diabetes patients
- The trial has an 8 week run-in period where basal insulin is titrated
- At the end of the 8 week run-in period there is CGM data for 67 subjects during 14 days
- The CGM device used is Dexcom G4

The maximum frequency (which is also called band edge frequency) of BG in diabetes patients in daily life with a typical schedule of meals, exercise, sleep, and insulin injection is 0.9 $10^{-3}$ Hz (K.-D. K. B. T. Gough DA, "Frequency characterization of blood glucose dynamics. Ann Biomed Eng. 2003, 31: 91-97"). The intrinsic BG dynamics are therefore not faster than approximately 18 min (the inverse of the band edge frequency). So, missing data gaps shorter than roughly 20 min of consecutive data do not impact the safety of dose recommendation.

Based on these considerations a rule-set for actions based on missing CGM data can be proposed as set out in Table 1 above.

Of the 917 individual CGM data sets NN 1218-3853, 134 pertain to the first 2 days where there is not yet 3 consecutive days of CGM data, and thus no actions can be recommended. 16 data sets which do not contain CGM values are not included in the table below. Of the remaining 767 data sets, the recommendations are presented in Table 2, and should represent what can be expected on a continuous basis.

Table 2: Categorisation of the proposed actions based on NN1218-3853 CGM data

| Category (3 24-hour period measurements) | Frequency | Percentage |
|---|---|---|
| 1. No data gap interval larger than 20 min | 270 | 35 % |
| 2. There are gaps >20 min but <3 hours, and <60 5-minutes samples missing in any of the last 3 days | 317 | 41 % |
| 3. 1 day has gap of > 3 hours or > 60 5-min samples missing | 149 | 19 % |
| 4. > 1 day with gaps of > 3 hours or > 60 5-min samples missing | 31 | 4 % |

Overall, 76 % of the time the engine would provide dose recommendations based on 3 24 hour periods of CGM data, 19 % of the time the app would provide dose recommendations based on 2 24 hour periods of CGM data, and only in 4 % of the time would the app omit providing a dose recommendation based on the amount of missing CGM data.

Having described a specific example of how a dose guidance request is received and processed when stored instructions are executed, the issue of providing improved systems and methods for distributed resource management of computationally intensive tasks within a cloud computing environment in relation to medication dosing guidance will be addressed.

The proposed solution is to break up the backend engine and its functions into containers within a distributed microservice architecture. Containers, or otherwise known as operating-system-level virtualization, are a lightweight approach to virtualization that only provides the bare minimum that an application requires to run and function as intended.

In a way, they can be considered as super minimalist virtual machines that are not running on a hypervisor. By Containerizing each sub function of the engine, it enables it to run reliably in different environments by abstracting away the operating system and the physical infrastructure. The size of the containers is usually measured in tens of megabytes (whereas Virtual Machines are several GBs) and it only takes 1-2 seconds to provision one to meet additional demand. When the load is increasing new virtualizations can be created as needed and when the load drops containers can be destroyed. An orchestrator shall be included in the network to determine which function embedded in a container to execute and subsequently to then have any additional computing resources dedicated to it in order to meet the needs of the dose guidance system and to avoid a system time out.

This model ensures that computational resources are devoted on an individual function level rather than for the entire engine, ensuring the most efficient use of resources while minimizing computational waste and ultimately excess cost.

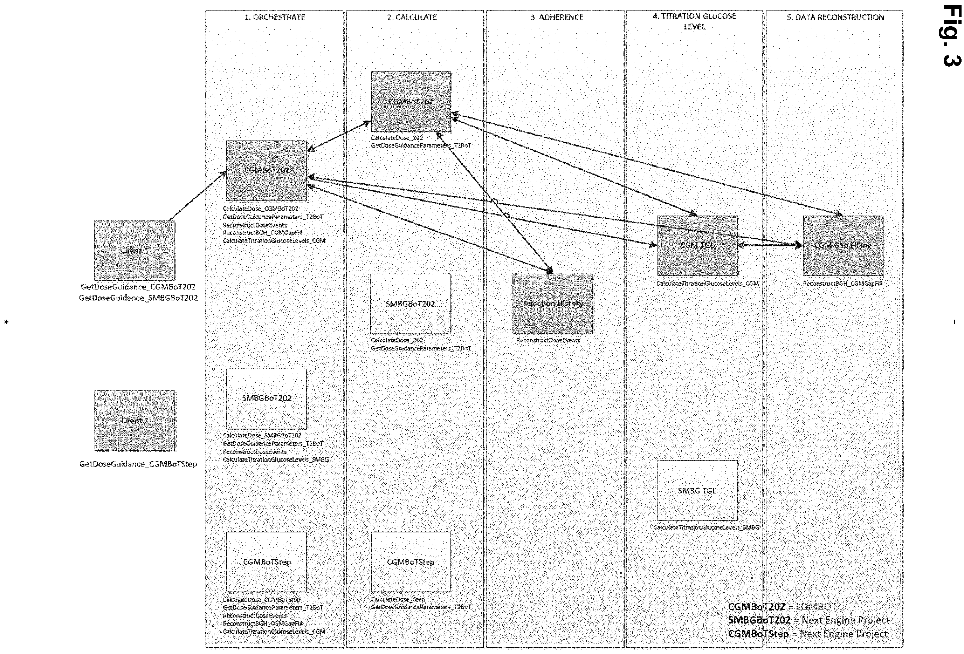

As illustrated in fig. 3 the shown engine is comprised of containers with embedded functions that communicate amongst one another via APIs (Application Programming Interface) in accordance with a microservice architecture at the calling of an orchestrator. The engine functions are separated into containers based upon the level of computing intensiveness as well as their level of reusability across different dose guidance systems. For example, the "2. Calculate Function" requires very little computing resources as it consists of a very simple mathematical function (e.g. addition/subtraction of a just a few numbers) as opposed to "5. Data Reconstruction" which may take thousands of data points at one time in order to do a regression analysis and interpolation in order to reconstruct a data set. It is up to the "1. Orchestrator" to identify which function is needed and when, which may trigger the any additional computational resources on an as needed basis. By this method, "5. Data Reconstruction" can be scheduled before or in parallel to "3. Adherence" as they can be executed independently of one another and "5. Data Reconstruction" can have additional resources dedicated to it thereby ensuring the response to the client doesn't time out.

As shown in fig. 3 a first orchestrator "CGMBoT202" has been provided for a first engine with 4 associated containers in 4 corresponding container classes. The set-up allows additional orchestrators, e.g. for engine "SMBGBot202" to be implemented which may reuse already implemented functions or may require new functions to be implemented in a corresponding container class. Indeed, new container classes may be needed for future types of functions.

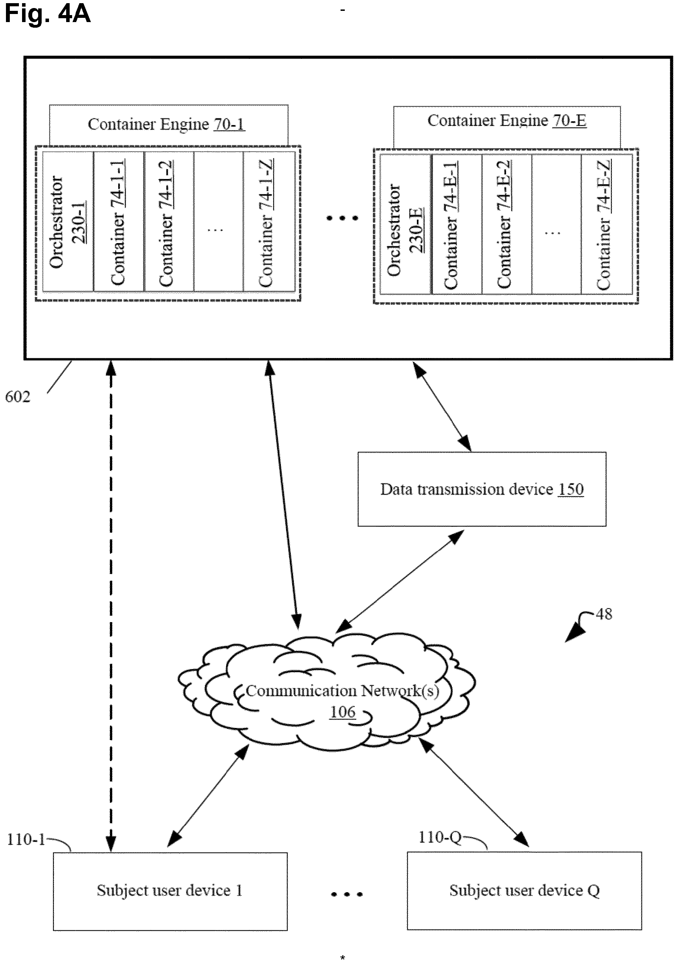

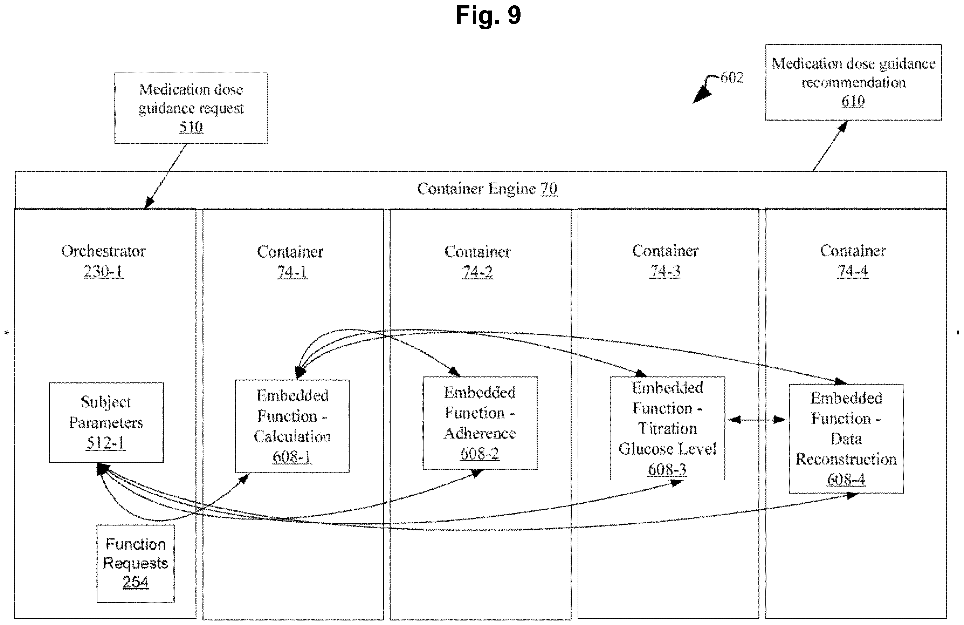

Fig. 4A illustrates an example of an integrated system 48, which depends on system 602 (variously "computer system 602," "medication dose guidance computing system 602," or "medication dose guidance system 602") for the determination of medication dose guidance recommendations for subjects. Fig. 9 provides more details of system 602, which illustrates the processing of medication dose guidance requests. The computer system 602 includes a container engine 70, an orchestrator 230, and one or more containers 74. A container is a user-space instances. Such user-space instances look like real computers from the point of view of functions that run on them. A computer program running on an ordinary operating system can see all resources (connected devices, files and folders, network shares, CPU power, quantifiable hardware capabilities) of that computer. However, programs (e.g., the disclosed functions) running inside a container can only see the container's contents and devices assigned to the container. Examples of containers includes, but are not limited to chroot, Docker, Linux-VServer, Imctfy, LXC, Singularity, OpenVZ, Vituozzo, Solaris Containers, FreeBSD jail, sysjail, WPARs, iCore Virtual Accounts, Sandboxie, Systemd-nspawn, and Turbo. See, Hogg, "Software Containers: Used More Frequently than Most Realize," Network World, May 26, 2016.

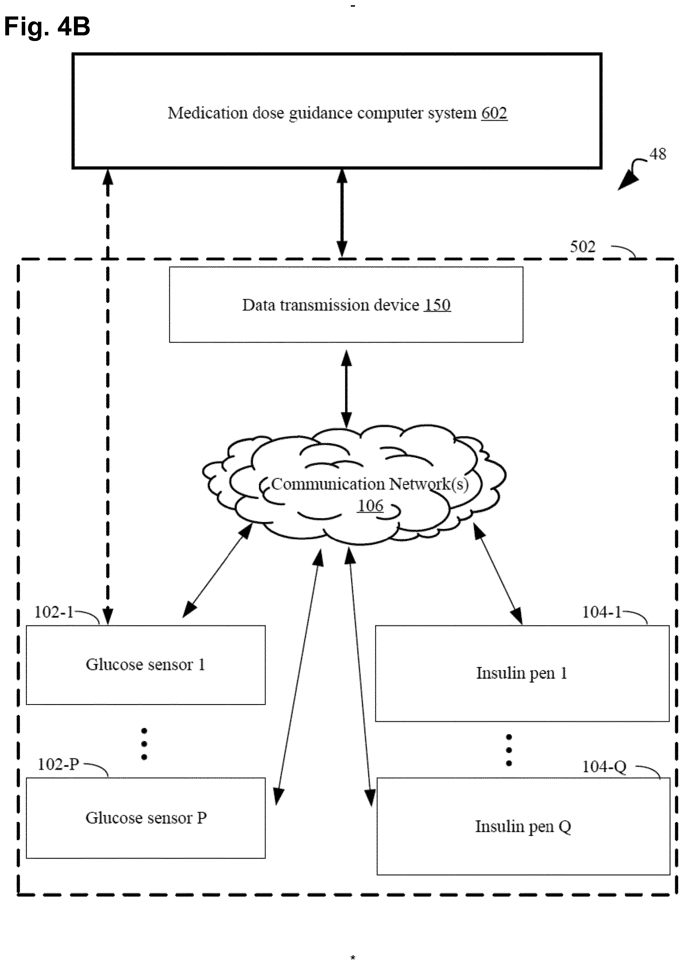

Fig. 4B illustrates a particular instantiation of system 48, where the subject user devices (e.g. 110-1) consist of glucose sensors 102 and insulin pens 104. The integrated system 502 includes one or more connected insulin pens 104, one or more glucose monitors 102, memory 506, and a processor (not shown) for performing algorithmic categorization of autonomous glucose data of a subject and requests for medication dose guidance recommendations. In some embodiments, glucose monitor 102 is a continuous glucose monitor.

Reference will now be made in detail to embodiments, examples of which are illustrated in the accompanying drawings. In the following detailed description of implementations, numerous specific details are set forth in order to provide a thorough understanding of the present invention. However, it will be apparent to one of ordinary skill in the art that the present invention may be practiced without these specific details.

Definitions

The terminology used in the present disclosure is for the purpose of describing particular embodiments only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes" and/or "comprising," when used in this specification, specify the presence of stated features, integers, steps, operations, elements, and/or components, but do not preclude the presence or addition of one or more other features, integers, steps, operations, elements, components, and/or groups thereof.

It will also be understood that, although the terms first, second, *etc.* may be used herein to describe various elements, these elements should not be limited by these terms. These terms are only used to distinguish one element from another. For example, a first filter could be termed a second filter, and, similarly, a second filter could be termed a first filter, without departing from the scope of the present disclosure. The first filter and the second filter are both filters, but they are not the same filter.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

The term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A, X employs B, or X employs both A and B. In addition, the articles "a" and "an" as used in this application and the appended claims should generally be construed to mean "one or more" unless specified otherwise or clear from the context to be directed to a singular form.

The terms "subject," "individual," and "user" are used interchangeably herein and refer to humans. Preferably, the individual is an adult individual.

As used herein, the term "medicament" refers to a chemical substance used for medical treatment of a subject. The terms "medicament," "medication," and "drug" are used interchangeably herein. The medicament may be prescribed by a physician or other health care provider. Alternatively, the medicament may be an over-the-counter drug or product. In a specific example of the present disclosure, the medicament is insulin, which is used to treat diabetes mellitus.

The term "diabetes" or "diabetes mellitus" includes type 1 diabetes mellitus, type 2 diabetes mellitus, gestational diabetes (during pregnancy) and other states that cause hyperglycemia. The term is used for a metabolic disorder in which the pancreas produces insufficient amounts of insulin, or in which the cells of the body fail to respond appropriately to insulin thus preventing cells from absorbing glucose. As a result, glucose builds up in the blood and treatment is required to control blood glucose levels.

According to the present invention, the insulin comprises or consists of long-acting insulin and ultra-long acting insulin. In principle, the longer the half-life of the insulin, the more stable and evenly distributed the glucose-lowering effect over a dosing interval (i.e. time interval between injections). According to the present invention, the insulin is administered in an amount to achieve a beneficial glycemic control in said subject. According to the present invention, the beneficial glycemic control in said subject is determined by at least the levels of $HbA_{1c}$ (glycosylated hemoglobin) in said subject after administration of said basal insulin. By use of the basal insulin and its administration according to the present invention it is possible to achieve improvements in the proportion of patients in need thereof reaching $HbA_{1c}$ targets.

By the term insulin pen is meant an injection device suitable for applying discrete doses of an insulin formulation, and wherein the injection device is also adapted for logging and communicating dose related data.

As used herein, the terms "component" and "system," as well as various forms thereof (e.g., components, systems, sub-systems, etc.) are intended to refer to a computer-related entity, either hardware, a combination of hardware and software, software, or software in execution. For example, a component may be, but is not limited to being, a process running on a processor, a processor, an object, an instance, an executable, a thread of execution, a program, and/or a computer. By way of illustration, both an application running on a computer and the computer can be a component. One or more components may be distributed in an extensible, cloud-based service. One or more components may reside within a process and/or thread of execution and a component may be localized on one computer and/or distributed between two or more computers.

Description

A detailed description of a system 48 for autonomously calculating a medicament dosage in a prescribed drug regimen for a subject, in accordance with the present disclosure, is described in conjunction with figs. 1 and 2. As such, figs. 1 and 2 collectively illustrate the topology of the system in accordance with the present disclosure. In the topology, there is a medication dose guidance system for autonomously adjusting a medicament dosage in a prescribed drug regimen for a subject ("medication dose guidance computer system 602") (figs. 1 and 2), a device for collecting information from a subject ("subject user device 110"), and a device for transmitting information between the medication dose guidance computer system 602 and the subject user device 110 ("data transmission device 150").

Referring to fig. 4A, the computer system 602 autonomously provides an adjusted a medicament dosage for a subject. Computer system 602 or portions thereof may include information obtained from a service (e.g. in the cloud) or may operate substantially in a cloud computing environment. As described for example in U.S. Patent Application 2017/0063833, entitled "Application Service Architecture," assigned to Microsoft Technology Licensing LLC, a cloud computing environment provides for information to reside on multiple devices in a network.

In some embodiments, the medicament is a drug prescribed by a health care provider. In some embodiments, the medicament is an over-the-counter drug. To calculate a medicament dose guidance recommendation, the subject user device 110, which is in electrical communication with the computer system 602 (either through data transmission device 150, through a communication network 106, or directly), receives autonomous subject-specific information related to the subject's usage of the medicament. For instance, in some embodiments the data transmission device 150 receives this data wirelessly through radio-frequency signals. In some embodiments such signals are in accordance with an 802.11 (WiFi), Bluetooth, or ZigBee standard. In some embodiments, the data transmission device 150 receives such data directly and transmits the data to the computer system 602, which then analyzes the data and provides a medication dose guidance recommendation (either directly to the subject user device 110 or through the data transmission device 150). In some embodiments, a subject user device 110 includes an RFID tag and communicates to the data transmission device 150 and/or the medication dose guidance computer system 602 using RFID communication. In some embodiments, the data transmission device 150 and/or the computer system 602 also obtains or receives physiological measurements of the subject (e.g., from wearable physiological measurement devices, from measurement devices within the data transmission device 150 such as a magnetometer or thermostat, etc.).

In some embodiments, the data transmission device 150 and/or the computer system 602 is not proximate to the subject and/or does not have wireless capabilities or such wireless capabilities are not used for the purpose of acquiring glucose data, insulin medicament injection data, and/or physiological measurement data. In such embodiments, a communication network 106 may be used to communicate subject information from the subject user device 110 to the data transmission device 150 and/or the medication dose guidance computer system 602 and/or physiological measurement data from one or more physiological measurement devices (not shown) to the data transmission device 150 and/or the medication dose guidance computer system 602.

Referring to fig. 4B, in some embodiments, the subject user device 110 consists of a glucose sensor 102 and/or an insulin pen 104. In some embodiments, the medication dose guidance computer system 602 and/or the data transmission deice 150 receives glucose measurements and insulin titration requests originating from one or more glucose sensors 102 attached to a subject on an ongoing basis. In some embodiments, the data transmission device 150 also receives insulin medicament injection data from one or more insulin pens 104 used by the subject to inject insulin medicaments. In some embodiments, the data transmission device 150 receives such data directly from the glucose sensor(s) 102 and insulin pens 104 used by the subject. In some embodiments, a glucose sensor 102 and/or insulin pen 104 includes an RFID tag and communicates to the data transmission device 150 and/or the computer system 602 using RFID communication. For some embodiments of the present invention, subject user device 110 is an insulin pen device. In some embodiments, the insulin pen device is FlexPen®(s) or FlexTouch®(s). FlexPen® and or FlexTouch® are trademarks of Novo Nordisk A/S.

In some embodiments, as shown in fig. 4B, the data transmission device 150 and/or the medication dose guidance computer system 602 is not proximate to the subject and/or does not have wireless capabilities or such wireless capabilities are not used for the purpose of acquiring glucose data, insulin medicament injection data, and/or physiological measurement data. In such embodiments, a communication network 106 may be used to communicate glucose measurements from the glucose sensor 102 to the data transmission device 150 and/or the medication dose guidance computer system 602, insulin medicament injection data from the one or more insulin pens 104 to the data transmission device 150 and/or the medication dose guidance computer system 602, and/or physiological measurement data from one or more physiological measurement devices (not shown) to the data transmission device 150 and/or the computer system 602.

Examples of networks 106 (as shown in fig. 4A and fig. 4B) include, but are not limited to, the World Wide Web (WWW), an intranet and/or a wireless network, such as a cellular telephone network, a wireless local area network (LAN) and/or a metropolitan area network (MAN), and other devices by wireless communication. The wireless communication optionally uses any of a plurality of communications standards, protocols and technologies, including but not limited to Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (e.g., IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (e.g., Internet message access protocol (IMAP) and/or post office protocol (POP)), instant messaging (e.g., extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS), or any other suitable communication protocol, including communication protocols not yet developed as of the filing date of the present disclosure.

In some embodiments, the data transmission device 150 is part of a subject user device 110. That is, in some embodiments, the data transmission device 150 and a subject user device 110 are a single device. In some embodiments, there is a single glucose sensor 102 attached to the subject and the data transmission device 150 is part of a glucose sensor 102. That is, in some embodiments, the data transmission device 150 and a glucose sensor 102 are a single device.

Of course, other topologies of the system 48 are possible. For instance, rather than relying on a communications network 106, the one or more subject user devices 110 may wirelessly transmit information directly to the data transmission device 150 and/or medication dose guidance computer system 602. Similarly, the one or more glucose sensors 102 and the one or more insulin pens 104 may wirelessly transmit information directly to the data transmission device 150 and/or medication dose guidance computer system 602. Further, the data transmission device 150 and/or the medication dose guidance computer system 602 may constitute a portable electronic device, a server computer, or in fact constitute several computers that are linked together in a network or be a virtual machine in a cloud computing context. As such, the exemplary topology shown in figs. 1A and 1B merely serve to describe the features of example embodiments of the present disclosure in a manner that will be readily understood to one of skill in the art.

While the system 48 disclosed in figs. 1A and 1B can work standalone, in some embodiments it can also be linked with electronic medical records to exchange information in any way.

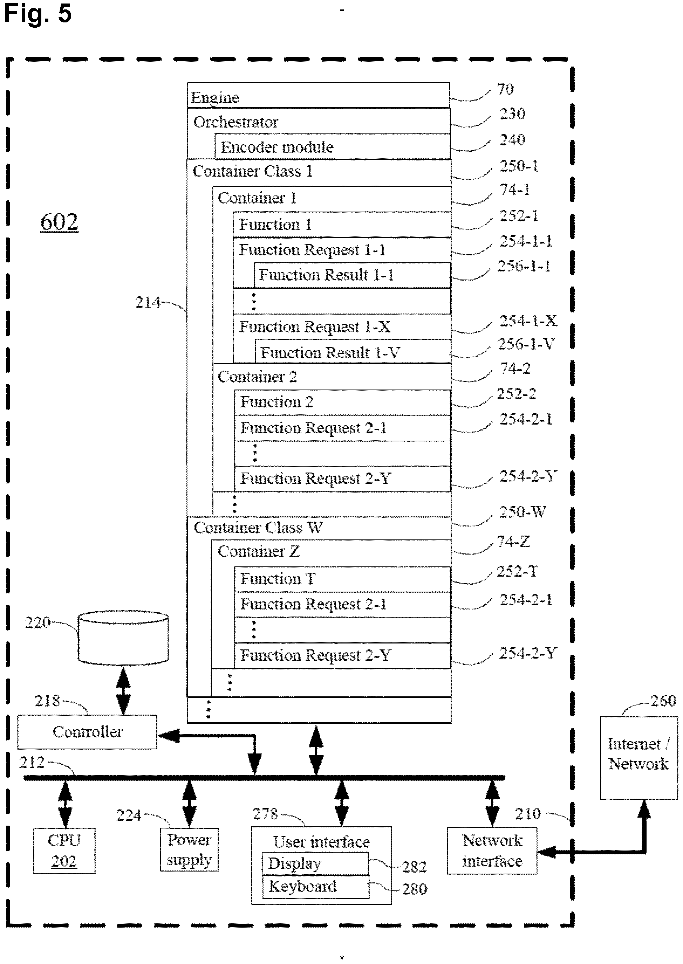

As illustrated in fig. 5, the computer system 602 preferably comprises one or more engines 70 within a distributed micro-service architecture. The components illustrated in fig. 5 are exemplary and are not meant to be all-inclusive of all components that may be needed or included.

Furthermore, the number of components may differ in other embodiments without departing from the scope of aspects of the subject matter described herein

Referring to fig. 5, in some embodiments, the computer system 602 comprises one or more systems. In some embodiments, the functionality for autonomously providing a medication dose guidance recommendation in a prescribed medicament regimen for a subject is spread across any number of networked computers and/or resides on each of several networked computers at a remote location accessible across the communications network 106 and/or is hosted on one or more cloud-based environments. One of skill in the art will appreciate that any of a wide array of different computer topologies are used for the application and all such topologies are within the scope of the present disclosure.

In some embodiments, an exemplary medication dose guidance computer system 602 for autonomously providing a medicament dosage recommendation in a prescribed medicament regimen for a subject comprises one or more processing units (CPU's) 202, a network or other communications interface 260, a memory 214 (*e.g.*, random access memory), one or more magnetic disk storage and/or persistent devices 220 optionally accessed by one or more controllers 218, one or more communication busses 212 for interconnecting the aforementioned components, and a power supply 224 for powering the aforementioned components. In some embodiments, data in memory 214 is seamlessly shared with non-volatile memory 220 using known computing techniques such as caching. In some embodiments, memory 214 and/or memory 220 includes mass storage that is remotely located with respect to the central processing unit(s) 202. In other words, some data stored in memory 214 and/or memory 220 may in fact be hosted on computers that are external to the medication dose guidance computer system 602 but that can be electronically accessed by the medication dose guidance computer system 602 over an Internet, intranet, or other form of network or electronic cable (illustrated as element 106 in fig. 4A) using network interface 210.

In some embodiments, the memory 214 of the medication dose guidance computer system 602 for providing medication dosing guidance recommendations to a plurality of subjects stores:

- an engine 70 that comprises one or more orchestrators 230 and a plurality of containers 74 (e.g. 74-1, 74-2, 74-Z),
- one or more orchestrators 230 that organize the subject data, the required functions for determining a medication dose guidance request, and the distribution of function requests 254 to containers 74,
- an encoder module 240 that embeds functions 252 into containers 74, and
- one or more containers 74, each of which contains embedded functions 252 and execute function requests 254.

In some embodiments, the medication dose guidance system 602 is accessible via any browser (phone, tablet, laptop/desktop). In preferred embodiments, the medication dose guidance system 602 is stored in a cloud computing environment.

In some implementations, one or more of the above identified data elements or modules of the medication dose guidance computer system 602 for providing medication dosing guidance recommendations to a plurality of subjects using a multi-function dose guidance algorithm comprising a plurality of functions are stored in one or more of the previously described memory devices, and correspond to a set of instructions for performing a function described above. The above-identified data, modules or programs (e.g., sets of instructions) need not be implemented as separate software programs, procedures or modules, and thus various subsets of these modules may be combined or otherwise re-arranged in various implementations. In some implementations, the memory 214 and/or 220 optionally stores a subset of the modules identified above. Furthermore, in some embodiments the memory 214 and/or 220 stores additional modules not described above.

In some embodiments, a medication dose guidance request for autonomously adjusting a long acting insulin medicament dosage 216 in a prescribed insulin regimen 212 for a subject is generated by a user device 278 such as a smartphone (e.g., an iPhone), laptop, tablet computer, desktop computer, or other form of electronic device (e.g., a gaming console). In some embodiments, the computer system 602 has any or all of the circuitry, hardware components, and software components found in the computer system 602 depicted in fig. 5. In the interest of brevity and clarity, only a few of the possible components of the computer system 602 are shown in order to better emphasize the additional software modules that may be installed on the computer system 602.

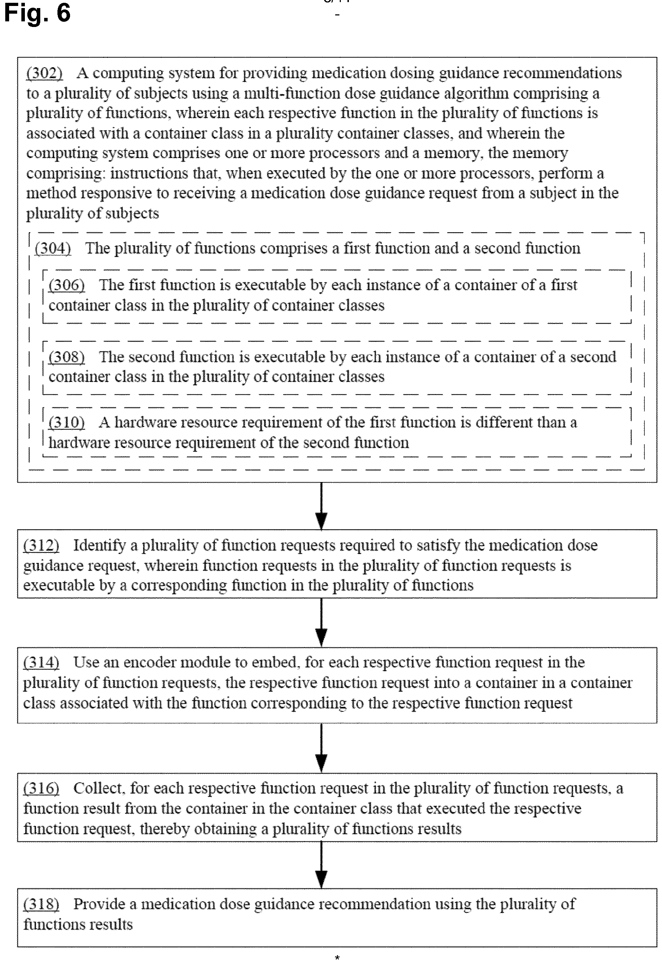

Now that details of a system 48 and device 200 for providing medication dosing guidance recommendations to a plurality of subjects using a multi-function dose guidance algorithm comprising a plurality of functions have been disclosed, details regarding a flow chart of processes and features of the system, in accordance with various embodiments of the present disclosure, are disclosed with reference to fig. 6.

With reference to block 302 in fig. 6, a goal of the present disclosure is to provide medication dosing guidance recommendations to a plurality of subjects using a multi-function dose guidance algorithm comprising a plurality of functions, in conjunction with a device such as the data transmission device 150 and a computer system 602. Each of the subjects has a diabetic condition.

dL In some embodiments this diabetic condition is type 2 diabetes mellitus. According to the invention, the medication dose guidance request is for a dose of insulin medicament, where the dose of insulin medicament is to achieve a predetermined blood glucose target range of a subject to treat diabetes mellitus.

In some embodiments, the predetermined blood glucose target range is 50-180mg/dL, 60-180mg/dL, 70-180mg/dL, 80-180mg/dL, 50-200mg/dL, 60-200mg/dL, 70-200mg/dL, 80-200mg/dL, 50-250mg/dL, 60-250mg/dL, 70-250mg/dL, or 80-250mg/dL.

In some embodiments, the plurality of subjects comprises at least 100 subjects, at least 1,000 subjects, at least 5,000 subjects, at least 10,000 subjects, at least 50,000 subjects or at least 100,000 subjects. In some embodiments, the system concurrently executes a medication dose guidance request for each such subject.

Each respective function 252 in the plurality of functions is associated with a container class 250 in a plurality of container classes. As illustrated in fig. 5, the computer system 602 includes one or more processors 202 and a memory 214/220. The memory includes instructions that, when executed by the one or more processors, perform a method responsive to receiving a medication dose recommendation request from a subject in the plurality of subjects.

In some embodiments, a container 74 in the container class 250 associated with a function 252 corresponding to the respective function request 254 evaluates whether the respective function request 254 contains input required for the function to provide a function result 256. In such instances, when the function request fails to contain the input required, no function result 256 is provided by the container in the container class associated with the function.

In some embodiments, the present disclosure is used to treat subjects that are at least 5 years old, at least 10 years old, at least 11 years old, at least 12 years old, at least 13 years old, at least 14 years old, at least 15 years old., at least 16 years old, at least 17 years old, at least 18 years old, at least 19 years old, or at least 20 years old.

In some embodiments, the present disclosure is used to treat subject whose body mass index is no greater than 32 kg/m$^2$. In some embodiments, the present disclosure is used to treat subject whose body mass index is no greater than 34 kg/m$^2$. In some embodiments, the present disclosure is used to treat subject whose body mass index is no greater than 35 kg/m$^2$. In some embodiments, the present disclosure is used to treat subject whose body mass index is no greater than 36 kg/m$^2$. In some embodiments, the present disclosure is used to treat subject whose body mass index is no greater than 37 kg/m$^2$. In some embodiments, the present disclosure is used to treat subject whose body mass index is no greater than 38 kg/m$^2$.

In some embodiments, the present disclosure is used to treat subjects whose body mass index is about 25 kg/m$^2$. In some embodiments, the present disclosure is used to treat subjects whose body mass index is between 20 kg/m$^2$ and 30 kg/m$^2$.

In some embodiments, the present disclosure is used to treat subjects that have been suffering from diabetes for at least 1 year, at least 5 years, at least 7 years, at least 9 years, or at least 11 years.

In some embodiments, the present disclosure achieves a baseline HbA$_{1c}$ level for subjects of no more than 7% after a number of weeks of treatment. In some embodiments, the present disclosure achieves a baseline HbA$_{1c}$ level for subjects of no more than 7% after a between 10 and 40 weeks of treatment, between 15 and 30 weeks of treatment, or between 20 and 28 weeks of treatment, or after 26 weeks of treatment. In some embodiments, the present disclosure achieves a baseline HbA$_{1c}$ level for subjects of no more than 6%, no more than 7%, or no more than 8% after a number of weeks of treatment. In some embodiments, the present disclosure achieves a baseline HbA$_{1c}$ level for subjects of no more than 6%, no more than 7%, or no more than 8% after between 10 and 40 weeks of treatment, between 15 and 30 weeks of treatment, or between 20 and 28 weeks of treatment, or after 26 weeks of treatment.

In some embodiments, the medicament comprises an insulin that is delivered by injection, such as by use of an insulin pen device 104. In some embodiments, the medicament is LysB29(Nc-hexadecandioyl-y-Glu) des(B30) human insulin (insulin degludec, Tresiba®).

According to the present invention, the basal insulin comprises or consists of long-acting insulin and ultra-long acting insulin.

In some embodiments, the medicament comprises a derivative or analogue of a naturally occurring insulin that:

(a) exhibits in physiological conditions, at least in part, the insulin receptor binding of the naturally occurring insulin, preferably, at least 0.01% of the insulin receptor binding of the naturally occurring insulin, for example, at least 0.1%, at least, 1%, at least 5%, at least 10%, at least 15%, at least 20%, at least 25% at least 50%, at least 65%, at least 75%, at least 85%, at least 95%, at least 100%, at least 110%, at least 120%, at least 130%, at least 140% or at least 150% of the insulin receptor binding of the naturally occurring insulin, and/or, at least in part, the potency of the naturally occurring insulin, preferably, at least 25% of the potency of the naturally occurring insulin, for example, at least 50%, at least 65%, at least 75%, at least 85%, at least 95%, at least 100%, at least 110%, at least 120%, at least 130%, at least 140% or at least 150% of the potency of the naturally occurring insulin, and
(b) exhibits a mean terminal half-life of at least 5 hours and less than 18 hours in physiological conditions when injected subcutaneously, for example, at least 7 hours, at least 8 hours, at least 10 hours, at least 12.5 hours, greater than 12.5 hours, at least 15 hours or at least 17.5 hours and less than 18 hours, between 5 and 17.5 hours, between 10 and 17.5 hours or between 15 and 17.5 hours.

In some embodiments, the medicament comprises a 'long-acting insulin' that also:
(c) induces in a subject a maximum deviation from mean insulin concentration (AUCF%) over a 24 hour period of $\leq \pm$ 20, for example $\leq \pm$ 18, $\leq \pm$ 17, $\leq \pm$ 16, $\leq \pm$ 15, $\leq \pm$ 14, $\leq \pm$ 13, $\leq \pm$ 12, $\leq \pm$ 11, $\leq \pm$ 10, $\leq \pm$ 9, $\leq \pm$ 8, $\leq \pm$ 7, $\leq \pm$ 6, $\leq \pm$ 5, $\leq \pm$ 4, $\leq \pm$ 3, $\leq \pm$ 2, $\leq \pm$ 1, $\leq \pm$ 0.5, $\leq \pm$ 0.1.

In some embodiments, the medicament comprises a 'long-acting insulin' comprising a derivative or analogue of a naturally occurring insulin that:

(a) exhibits in physiological conditions, at least in part, the insulin receptor binding of the naturally occurring insulin, preferably, at least 0.01% of the insulin receptor binding of the naturally occurring insulin, for example, at least 0.1%, at least, 1%, at least 5%, at least 10%, at least 15%, at least 20%, at least 25% at least 50%, at least 65%, at least 75%, at least 85%, at least 95%, at least 100%, at least 110%, at least 120%, at least 130%, at least 140% or at least 150% of the insulin receptor binding of the naturally occurring insulin, and/or, at least in part, the potency of the naturally occurring insulin, preferably, at least 25% of the potency of the naturally occurring insulin, for example, at least 50%, at least 65%, at least 75%, at least 85%, at least 95%, at least 100%, at least 110%, at least 120%, at least 130%, at least 140% or at least 150% of the potency of the naturally occurring insulin,
(b) exhibits a mean terminal half-life of at least 18 hours in physiological conditions when injected subcutaneously, for example, greater than 18 hours, at least 20 hours, greater than 20 hours, greater than 22 hours, at least 22.5 hours, or greater than 24 hours, at least 25 hours, at least 27.5 hours, at least 30 hours, at least 32.5, at least 35 hours, at least 37.5 hours, or at least 40 hours, or between 18 and 40 hours, between 20 and 40 hours, between 24 and 40 hours.

In some embodiments, the medicament comprises a 'long-acting insulin' that also:
(c) induces in a subject a maximum deviation from mean insulin concentration (AUCF%) over a 24 hour period of $\leq \pm$ 20, for example, $\leq \pm$ 18, $\leq \pm$ 17, $\leq \pm$ 16, $\leq \pm$ 15, $\leq \pm$ 14, $\leq \pm$ 13, $\leq \pm$ 12, $\leq \pm$ 11, $\leq \pm$ 10, $\leq \pm$ 9, $\leq \pm$ 8, $\leq \pm$ 7, $\leq \pm$ 6, $\leq \pm$ 5, $\leq \pm$ 4, $\leq \pm$ 3, $\leq \pm$ 2, $\leq \pm$ 1, $\leq \pm$ 0.5, $\leq \pm$ 0.1.

In principle, the longer the half-life of the insulin, the more stable and evenly distributed the glucose-lowering effect over a dosing interval (i.e. time interval between injections).

According to the present disclosure, the basal insulin comprises or consists of long-acting insulin and ultra-long acting insulin.

According to the present disclosure, the basal insulin is administered in an amount to achieve a beneficial glycemic control in the subject.

In some embodiments, the medicament consists of a single insulin medicament having a duration of action that is between 12 and 24 hours or a mixture of insulin medicaments that collectively have a duration of action that is between 12 and 24 hours. Examples of such insulin medicaments include, but are not limited to, Insulin Degludec (developed by NOVO NORDISK under the brand name Tresiba®), NPH (Schmid, 2007, "New options in insulin therapy," J Pediatria (Rio J). 83(Suppl 5): S146-S155), Glargine (LANTUS, March 2, 2007), Insulin Glargine injection (Dunn et al. 2003, "An Updated Review of its Use in the Management of Diabetes Mellitus" Drugs 63: p. 1743), and Determir (Plank et al., 2005, "A double-blind, randomized, dose-response study investigating the pharmacodynamic and pharmacokinetic properties of the long-acting insulin analog detemir," Diabetes Care 28:1107-1112).

In some embodiments, the plurality of functions 252 comprises a first function and a second function (304). In some embodiments, the first function (e.g. 252-1) performs an addition or subtraction. In some embodiments, the second function (e.g. 252-2) performs a regression analysis and/or interpolation (e.g. to reconstruct a dataset). In some embodiments, the first function (e.g. 252-1) is executable by each instance of a container (e.g. 74-1) of a first container class (e.g. 250-1) in the plurality of container classes. In some embodiments, the container classes include a data reconstruction class, an adherence class, a calculate class, and/or a titration glucose level class. In some embodiments, an orchestrator 230 distributes function requests 254 to containers (e.g. 74-1 and 74-2) that are instantiations of the necessary container class (e.g. 250-1).

In some embodiments, each instance of a container (e.g. 74-2 and 74-2) of the first container class (e.g. 250-1) is hosted by server in a first set of servers (not shown) and each instance of a container (e.g. 74-Z) of the second container class (e.g. 250-W) is hosted by a server in second set of servers. In such embodiments, the first set of servers is a different than the second set of servers. In some embodiments, such a topology has the advantage of allowing for the provision of more powerful, or a larger set, of servers for containers that server more computationally intensive functions. In alternative embodiments, containers of two or more different classes are permitted to run concurrently on the same server.

Referring to block 306, in some embodiments the first function is executable by each instance of a container of a first container class in the plurality of container classes. For example, when the first function is a titration glucose level class, each instance of the container of the first container class has the titration glucose level class.

Referring to block 308, in some embodiments the second function is executable by each instance of a container of a second container class in the plurality of container classes. In other words, there is a one-to-one correspondence between respective functions offered by the computing system and respective container classes.

In some embodiments, the plurality of container classes comprises two or more container classes, three or more container classes, four or more container classes or five or more container classes.

Referring to block 310, in some embodiments a hardware resource requirement of the first function is different than a hardware resource requirement of the second function. In other words, in some embodiments, the plurality of functions comprises a first function and a second function, the first function is executable by each instance of a container of a first container class in the plurality of container classes, the second function is executable by each instance of a container of a second container class in the plurality of container classes, and a hardware resource requirement of the first function is different than a hardware resource requirement of the second function. In one such example, the first function is computationally more expensive than a second function. For instance, containers of the container class that handle the first function require more CPU time than containers of the container class that handle the second function. Alternatively, or additionally, containers of the container class that handle the first function may require more random-access memory than containers of the container class that handle the second function. As such, the relative resource requirements of containers are taken into consideration in some embodiments of the present disclosure when deciding whether to initiate another container of a specific container class for a given type of function. That is, the number of requests for a particular function is weighted (scaled, normalized) by an estimated measure of central tendency of the hardware resource requirements across requests of for the particular function have over time. In some embodiments, records are kept of the resource requirements of particular functions across historical requests for such functions in order to derive suitable metrics (scaling factors) for the relative resource requirements of each function in the plurality of functions offered by the disclosed systems. In some embodiments, a measure of central tendency of the resource requirements of such function requests are tracked on a function by function basis over time. In some embodiments, older function requests are down weighted relative to more recent function requests when computing such measures of central tendency.

In some embodiments, the encoder module further comprises evaluating each respective function for a resource requirement (e.g. as opposed to evaluating the resource requirements of an entire engine 70, thus ensuring the most efficient use of resources and minimizing computational waste). According to the invention, a composite resource requirement of a first set of instances of containers of a first container class is evaluated on a recurring basis. When the composite resource requirement of the first set of instances of containers of the first container class satisfies a first resource allocation threshold associated with the first set of containers (e.g. when more function requests of a corresponding function are being made than can be dealt with by the set of currently allocated container with the corresponding function), one or more additional instances of containers of the container class is added to the first set of containers and permitted to accept function requests from the encoder module that match the type of the first container class. In some embodiments, the first resource allocation threshold associated with a given container class is adjusted over time based on an evaluation of the measure of central tendency of the resource requirements of the respective serviced function requests of the given container class that are tracked over time as discussed above.

In some embodiments, the first resource allocation threshold associated with a given container class is satisfied when the random-access memory collectively used by a set of containers in the container class is $\geq$1MB, $\geq$5MB, $\geq$50MB, $\geq$500MB, $\geq$1GB, or $\geq$5GB. In some embodiments, the first resource allocation threshold associated with a given container class is satisfied when a set of containers in the container class requires $\geq$0.05 seconds, $\geq$0.5 seconds, $\geq$1 second, $\geq$5 seconds, $\geq$10 seconds, $\geq$15 seconds, $\geq$30 seconds, $\geq$1 minute, or $\geq$5 minutes, on average, to return a function result 256.

According to the invention, when the composite resource requirement of the first set of instances of the first container class satisfies a second resource allocation threshold associated with the first set of containers, one or more containers in the first set of containers is removed from the first set of containers and no longer permitted to accept function requests from the encoder module. In some embodiments, the second resource allocation threshold associated with a given contain class is adjusted over time based on an evaluation of the measure of central tendency of the resource requirements of the respective serviced function requests of the given container class that are tracked over time as discussed above.

In some embodiments, the second resource allocation threshold associated with a given container class is satisfied when the collective random-access memory used for a set of containers in the container class is ≤1MB, ≤5MB, ≤50MB, ≤500Mb, ≤1GB, or ≤5GB. In some embodiments, the second resource allocation threshold associated with a given container class is satisfied when a set of containers in the container class requires ≤0.05 seconds, ≤0.5 seconds, ≤1 second, ≤5 seconds, ≤10 seconds, ≤15 seconds, ≤30 seconds, ≤1 minute, or ≤5 minutes, on average, to return a function result 256.

Referring to block 312 of fig. 6, in some embodiments the method identifies a plurality of function requests required to satisfy the medication dose guidance request for a particular subject. In such embodiments, the function requests 254 in the plurality of function requests is executable by a corresponding function in the plurality of functions. In some embodiments, the plurality of function requests includes request for two or more functions that require the service of two or more corresponding container classes. In some embodiments, the plurality of function requests includes request for three or more functions that require the service of three or more corresponding container classes. In some embodiments, the plurality of function requests includes request for four or more functions that require the service of four or more corresponding container classes. In some embodiments, the plurality of function requests includes request for five or more functions that require the service of five or more corresponding container classes.

In some embodiments the plurality of functions request includes a first function request. In some embodiments, this first function request is for calculating a medication dose guidance recommendation of the subject (e.g. providing a new and/or an updated dose recommendation to the subject). In alternative embodiments, this first function request is for reconstructing an insulin injection history of the subject. For instance, in some embodiments, the insulin injection history is collected by an insulin pen 104, which also transmits the insulin injection history of the subject to the medication dose guidance system 602. In some embodiments, the insulin injection history contains temporal gaps (e.g. missing data points) or other errors (e.g. a logging of a 'priming' event as an insulin injection). Such occurrences are described in United States Patent Application Publication No. 2018/0014776 A1 entitled "Patient Mounted Micro Vein Enhancer," filed August 23, 2017. In some embodiments, the medication dose guidance system 602 must reconstruct the insulin injection history of the subject as part of providing an insulin dose guidance recommendation.

In some embodiments, the first function request is for calculating a titration glucose level of the subject. The titration glucose level is based on the blood glucose history, collected by a glucose sensor 102 of the subject and the insulin injection amounts administered by an insulin pen 104. The titration glucose level is specific to each subject and in some embodiments is also based on one or more subject parameters 512. In some embodiments, the method of calculating a titration glucose level is as described in International Application No. PCT/EP2017/065578 entitled "Basal Titration with Adaptive Target Glucose Level," filed June 23, 2017.

In some embodiments, the first function request is for reconstructing a blood glucose history of the subject. In some embodiments, the blood glucose history of a subject is measured by a glucose sensor 102, which in some embodiments is a continuous blood glucose monitor such as that described by United States Patent Publication No. 2004/0153257 A1, entitled "Graphical Display for Medical Devices and Methods for Displaying Medical Information," filed December 9, 2003. In some embodiments, the blood glucose history contains temporal gaps (e.g. missing data points). For instance, continuous blood glucose monitors may, due to user error or other technical difficulty in recording blood glucose levels, fail to record blood glucose levels of a subject for an intermittent period of time. In some embodiments, the glucose measurements are autonomously measured by the glucose sensor. For instance, in some embodiments the glucose sensor 102 is exemplified by the FREESTYLE LIBRE CGM by ABBOTT ("LIBRE") that makes autonomous glucose measurements of a subject. The LIBRE allows calibration-free glucose measurements with an on-skin coin-sized sensor, which can send up to eight hours of data to a reader device via near field communications, when brought close together. The LIBRE can be worn for fourteen days.

In some embodiments, the first function request is for obtaining dose guidance parameters of the subject (e.g. subject parameters 512). In some embodiments, dose guidance parameters include one or more of a body weight of the subject, the age of the subject, an upper limit target glucose range of the subject, a lower limit target glucose range of the subject, an overbasalisation limit of the subject, a most recent medication dose guidance recommendation and/or a starting insulin basal dose of the subject, and/or a basal injection history of the subject.

In some embodiments, the upper limit target glucose range used to determine the updated most recent medication dose guidance recommendation is within 80-180mg/dL, 90-180 mg/dL, 100-180mg/dL, 90-200mg/dL, 90-250mg/dL or 90-300mg/dL.

In some embodiments, the lower limit target glucose range used to determine the updated medication dose guidance recommendation is within 50-70mg/dL, 70-90 mg/dL, 70-100mg/dL, 60-100mg/dL, or 60-90mg/dL.

In some embodiments, the reconstructing a blood glucose history of a subject comprises calculating a reconstructed blood glucose history of the subject when the blood glucose history time course contains a temporal gap (e.g. if the subject's continuous glucose monitor has a sensor error - either from user or technical error - and does not record blood glucose measurements for an intermittent period of time or if the subject makes an error that affect the continuous glucose monitor). In some embodiments, a gap in the time course consists of a predetermined time period where no blood glucose measurements were recorded. In some embodiments, the predetermined time period defining a gap in the blood glucose history is ≥5 minutes, ≥10 minutes, ≥20 minutes, ≥30 minutes, or ≥1 hour.

In some embodiments, when there is a gap in the blood glucose history, the reconstructed blood glucose history is calculated based on the blood glucose history of the subject over past hour immediately preceding the gap, the past week immediately preceding the gap, the past two weeks immediately preceding the gap, the past month immediately preceding the gap, or the past year immediately preceding the gap. In some embodiments, the reconstructed blood glucose history is calculated based on the blood glucose history of the 1 minute before and after the gap, the 5 minutes before and after the gap, the 10 minutes before and after the gap, the 15 minutes before and after the gap, the 30 minutes before and after the gap, and/or the 1 hour before and after the gap.

In some embodiments, the input required by a function request comprises, for a given subject, (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and/or (vii) a basal injection history of the subject. Some function requests require only a subset of this information. Some function requests require additional data.

In some embodiments, the input required by a function request comprises, for a given subject, any one of (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and (vii) a basal injection history of the subject. Some such function requests require additional data.

In some embodiments, the input required by a function request comprises, for a given subject, any two of (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and (vii) a basal injection history of the subject. Some such function requests require additional data.

In some embodiments, the input required by a function request comprises, for a given subject, any three of (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and (vii) a basal injection history of the subject. Some such function requests require additional data.

In some embodiments, the input required by a function request comprises, for a given subject, any three of (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and (vii) a basal injection history of the subject. Some such function requests require additional data.

Referring to block 314, in some embodiments, the method uses an encoder module 240 to embed, for each respective function request 254 in the plurality of function requests, the respective function request 254 into a container in a container class associated with the function corresponding to the respective function request 254. In some embodiments, a given container in the container class associated with the function corresponding to the respective function request is concurrently services two or more, three or more, four or more, five or more, six or more, seven or more, eight or more, 10 or more, fifty or more, 100 or more, 1000 or more, or 10,000 or more requests of the same class for two or more, three or more, four or more, five or more, six or more, seven or more, eight or more, 10 or more, fifty or more, 100 or more, 1000 or more, or 10,000 subjects.

Referring to block 316, in some embodiments the method collects, for each respective function request 254 in the plurality of function requests, a function result 256 from the container in the container class that executed the respective function request 254, thereby obtaining a plurality of function results for a single subject in the plurality of subjects. Toward this end, in some embodiments, a first function and a second function for a subject are concurrently executed. In alternative embodiments, the first function and the second function for a subject are executed sequentially. In still other embodiments, Boolean logic is used to gate whether certain function requests are made for a given subject. For example, in some embodiments, a second function request corresponding to a second container class is not fired for a subject when a function result of a first function request corresponding to a first container class provides a result that fails to satisfy a monitoring condition.

Referring to block 318, in some embodiments the method provides a medication dose guidance recommendation using the plurality of function results. Block 318 is achieved when all the requested function requests return suitable function results that can be processed in order to compute a valid dosage recommendation for a subject. In some embodiments, the method further comprises performing a quality check of the dose guidance request, and wherein when the quality check fails (e.g. when the data quality check reveals that the function request lacks the necessary information - e.g. information regarding the subject parameters 512, or information regarding the required function - for the corresponding function to process the function request, or when the size of the function request would overwhelm the available computing resources), no medication dose guidance recommendation is provided. In some embodiments, when the data quality check fails and when either the subject records a hypoglycemic event or the subject has failed to take a previous recommended medication dose, a medication dose guidance recommendation is provided. In some embodiments, the subject records a hypoglycemic event when the blood glucose level of the subject is below 70mg/L, 75mg/L, 80mg/L, 65mg/L, 60mg/L, 55mg/L, 50mg/L, 45mg/L, or 40mg/L. In some embodiments, the subject fails to take a previous recommended medication dose when the subject has forgotten to take a dose or when the subject has taken an incorrect dose amount.

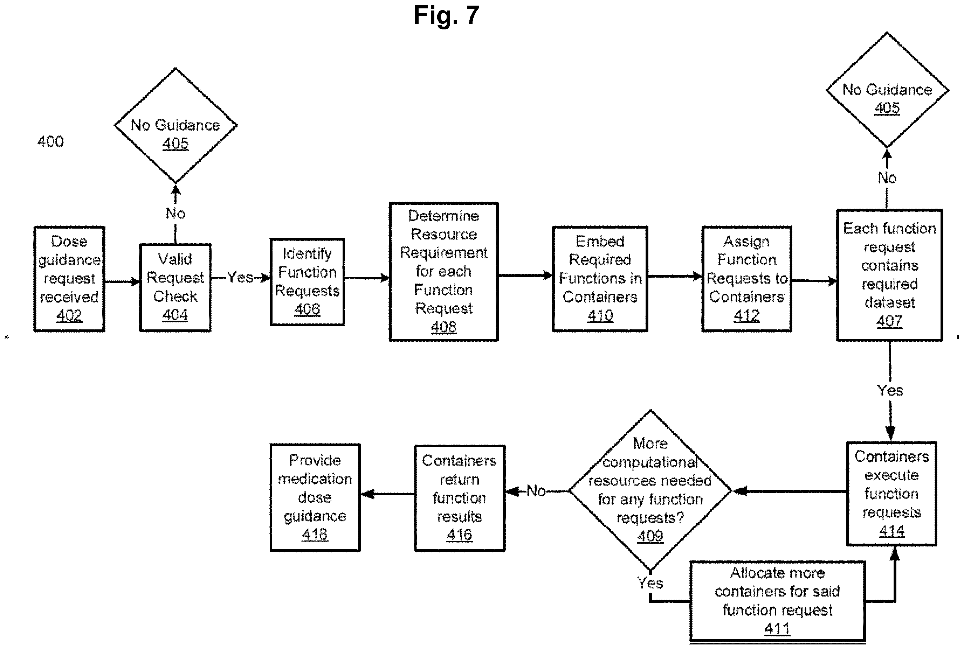

Fig. 7 illustrates an example method 400 (e.g. performed at an electronic device) for providing a medication dose guidance recommendation to the subject. In some embodiments, the method of fig. 7 is performed when the subject has not previously been provided with a medication dose guidance recommendation. In some embodiments, the method of fig. 7 provides a re-recommendation of a previously provided medication dose guidance recommendation. In some embodiments, the method of fig. 7 is performed to provide an updated medication dose guidance recommendation, subsequent to providing one or more previous medication dose guidance recommendations.

Referring to fig. 7, the computer system 602 receives a medication dose guidance request 402. In some embodiments, the medication dose guidance request is automatically generated (without human intervention) by a user device (e.g. subject user device 110). In some embodiments, the user makes a specific request for medication dose guidance. The method proceeds and checks 404 that the request is valid (*e.g.* the system confirms that the requisite or required data is included in the request - e.g. one or more of a body weight of the subject, an upper target glucose range of the subject, a lower limit target glucose range of the subject, an over-basalisation of the subject, a most recent medication dose guidance recommendation and/or a starting basal insulin dose, a blood glucose history of the subject, a basal insulin injection history of the subject and/or an injection data refresh of the subject). In some embodiments, the input required by the function request 254 comprises, for a given subject, is any one or more of, any two or more of, any three or more, any four or more of, any five or more of any six or more, all seven of, at least all seven of: (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent medication dose guidance recommendation and/or a starting insulin basal dose of the subject, and (vii) a basal injection history of the subject. In some embodiments, the input required by the function request 254 comprises, for a given subject, is a subset of: (i) a body weight of the subject, (ii) an upper limit target glucose range of the subject, (iii) a lower limit target glucose range of the subject, (iv) an overbasalisation limit of the subject, (v) a blood glucose history of the subject, (vi) a most recent insulin titration recommendation and/or a starting insulin basal dose of the subject, and (vii) a basal injection history of the subject.

If the medication dose guidance request does not contain the necessary data, the method ends 405, provides no guidance and, optionally, advises the user to return later with the proper data. In some embodiments, the device advises the user what data is missing or suggests the user seek assistance with their medical devices. If the medication dose guidance request contains the appropriate information, the method identifies 406 the function requests 254 required to provide the requested medication dose guidance recommendation (*e.g.* the function requests contained within the dose guidance request), where each function request 254 in the plurality of function requests is executable by a corresponding function in the plurality of functions. The method makes an initial hardware resource requirement (*e.g.* memory requirements, computational complexity, *etc.*) determination 408 for each function request. In some embodiments, the method determines the number of containers required and/or the division of containers by types of function requests. In some embodiments, the orchestrator 230 performs the hardware resource determination. In some embodiments, the hardware resource determination includes instantiating containers of the container classes associated with the functions required to perform the function requests necessary to provide a medication dosage recommendation to the subject.

The method proceeds and embeds functions related to the function requests into the selected (e.g. the instantiated) containers (410). In some embodiments, the orchestrator 230 performs the embedding. The function requests are assigned 412 to containers with the appropriate embedded functions. Each function request 254 is evaluated 407 for containing the required dataset to perform the function. If the function request 254 does not contain the required dataset, the method ends the process 405, provides no guidance, and optionally advises the user to return later.

For each function request 254 that has been assigned to a container and contains the required dataset, the respective container executes 414 the function request. If the method determines 409 that more computational resources are needed for the system to perform a function request 254, more containers in the respective container class are allocated 411 to perform the respective function request. If the method determines that sufficient computational resources are available to execute a function request, the method proceeds.

The containers return 416 results to the function requests. In some embodiments, the containers execute the function requests for each dose guidance request 510 concurrently. In some embodiments, the containers execute the function requests for each dose guidance request 510 sequentially. In some embodiments, the containers execute a subset of the function requests for a dose guidance request 510 concurrently and another subset of function requests sequentially. The method proceeds and provides 418 the result (*e.g.* a medication dose guidance recommendation) to the subject using the plurality of function results (e.g. 256-1-1, 256-1-V, etc.). In some embodiments, the result is provided directly to the user (e.g. to the subject user device 110). In some embodiments, the result is provided via a data transmission device 150 to the user.

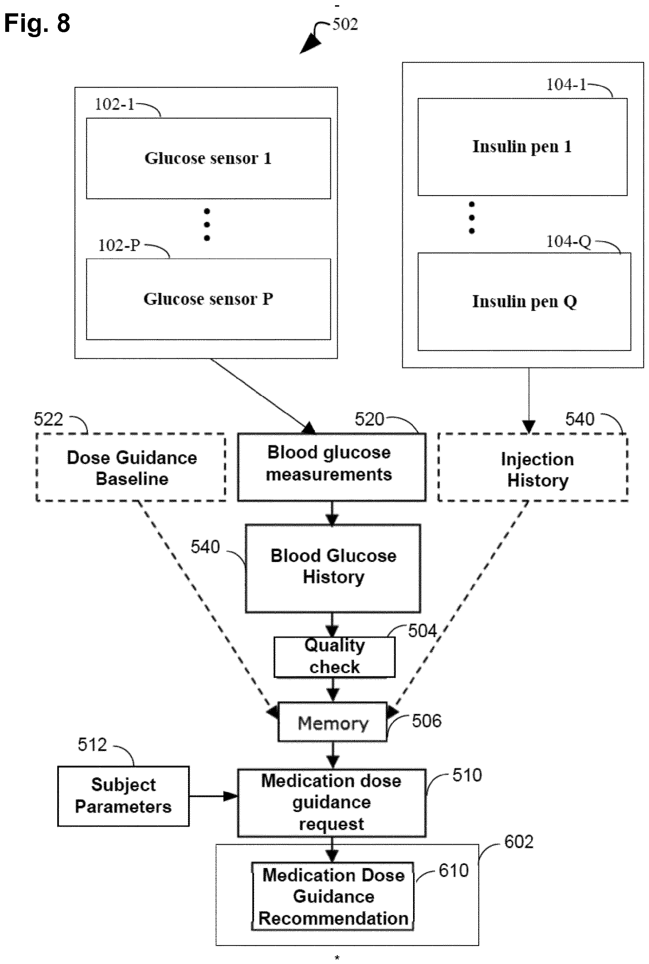

Referring to fig. 8, in some embodiments of the present disclosure, the medication dose guidance request 510 is a request for guidance on titrating an insulin dosage for the lowering of blood sugar for the treatment of diabetes mellitus. In such embodiments, the subject user device 110 may consist of one or more of a glucose sensor 102 and an insulin pen 104. The integrated system 502, autonomously obtains 520 timestamped insulin injection and blood glucose measurements of a subject (e.g. from a glucose sensor 102 and/or an insulin pen 104). Also, in some embodiments, data from the one or more insulin pens 104, which are used by the subject to apply a prescribed insulin regimen, is obtained 540 as a plurality of injection history records. Each injection history data point comprises a timestamped event specifying an amount of injected insulin medicament that the subject received as part of the prescribed insulin medicament dosage regimen. The glucose measurements (*e.g.*, the blood glucose history) are quality assessed 504, and a reconstructed blood glucose history is calculated when necessary. The blood glucose history or the reconstructed blood glucose history is stored in non-transitory memory 506. The memory 506 includes instructions that, when executed by the one or more processors, perform a method responsive to receiving a dose guidance request. A dose guidance recommendation request is sent 510 to the computer system 602 to provide a medication dose guidance recommendation 610, in accordance with the methods of the present disclosure.

In some embodiments, the medication dose guidance request includes obtaining subject parameters 512 that comprise at least (i) a body weight of the subject, (ii) an upper limit target glucose range of the subject, (iii) a lower limit target glucose range of the subject, and (iv) an overbasalisation limit of the subject. In some embodiments, the medication dose guidance request also includes obtaining a second data set 522 (the medication dose guidance baseline) that contains at least (i) a most recent medication dose guidance recommendation and/or (ii) a starting basal dose. In some embodiments, the medication dose guidance request also includes obtaining a blood glucose data set 520 that includes a plurality of glucose measurements of the subject taken over a time course to establish a blood glucose history and, for each respective glucose measurement in the plurality of glucose measurements, a corresponding glucose timestamp representing when in the time course the respective glucose measurement was made. In some embodiments, the medication dose guidance request also includes obtaining an injection history data set 540 comprising (i) a basal insulin injection history of the subject, wherein the injection history comprises a plurality of injections during all or a portion of the time course and, for each respective injection in the plurality of injections, (ii) a corresponding dose event amount and (iii) a dose event timestamp representing when in the time course the respective injection event occurred and where the second data set further comprises (iv) a last injection data refresh of the subject.

In some embodiments, the upper limit target glucose range used to determine the medication dose guidance recommendation is within 80-180mg/dL, 90-180 mg/dL, 100-180mg/dL, 90-200mg/dL, 90-250mg/dL or 90-300mg/dL. In some embodiments, the lower limit target glucose range is used to determine the medication dose guidance recommendation and is within 50-70mg/dL, 70-90 mg/dL, 70-100mg/dL, 60-100mg/dL, or 60-90mg/dL.

In some embodiments, the glucose measurements 522 are autonomously measured. The FREESTYLE LIBRE CGM by ABBOTT ("LIBRE") is an example of a glucose sensor that may be used as a glucose sensor 102 in order to make autonomous glucose measurements of a subject. The LIBRE allows calibration-free glucose measurements with an on-skin coin-sized sensor, which can send up to eight hours of data to a reader device (e.g., the data transmission device 150 and/or the computer system 602) via near field communications, when brought close together. The LIBRE can be worn for fourteen days in all daily life activities.

In some embodiments, the medication dose guidance request also includes evaluating the provided subject information for whether a set of evaluation information contains at least (i) the body weight of the subject, (ii) the plurality of glucose measurements of the subject taken over the time course, (iii) the injection history of the subject, (iv) the last medication dose guidance recommendation and/or the starting long-acting or ultra-long-acting insulin dose of the subject, (v) the overbasalisation limit of the subject, (vi) the last injection data refresh for the subject, (vii) the upper limit target glucose range of the subject, and (viii) the lower limit target glucose range of the subject. When a determination is made that the provided subject information fails to contain the required set of evaluation information, no medication dose guidance recommendation is made. When a determination is made that the appropriate evaluation information is available, the method of the computing system 602 comprises providing a medication dose guidance recommendation 610.

In some embodiments, the target blood glucose target range, the achievement and maintenance of which is a primary goal of insulin titration, is 50-180mg/dL, 60-180mg/dL, 70-180mg/dL, 80-180mg/dL, 50-200mg/dL, 60-200mg/dL, 70-200mg/dL, 80-200mg/dL, 50-250mg/dL, 60-250mg/dL, 70-250mg/dL, or 80-250mg/dL.

Insulin doses may be determined through a variety of methods, for example, they may be calculated based on weight (and/or height), fasting blood glucose and gender (thereafter adjusted empirically according to fast blood glucose and/or HbA$_{1c}$ level outcome). It is increasingly common to use a titration method wherein, after administering an initial dose standard or empirically-determined dose, further doses are adjusted by pre-determined increments (e.g. titration algorithm), as necessary, based on blood glucose/plasma measurements in order to reach and maintain a target blood glucose/plasma and/or HbA$_{1c}$ level. Such titration models are however always given as guidance only and individual adjustments (e.g. via an insulin titration guidance recommendation) are applicable on a case by case basis.

Referring to Fig. 9, the computing system 602 receives medication dose guidance requests 510 from one or more subjects and provides medication dose guidance recommendations 610 to each subject. In preferred embodiments, medication dose guidance system 602 resides in a cloud environment. The container engine 70 receives the medication dose guidance request 510. In some embodiments, the engine functions are separated into containers 74 based upon the level of computational intensiveness as well as the level of reusability of the containers across different dose guidance systems (e.g. how frequently a respective function in the plurality of function is used). The medication dose guidance request 510 contains subject information 512. In some embodiments, subject information 512-1 comprises at least a blood glucose history of the subject, a most recent insulin titration recommendation and/or a starting insulin basal dose of the subject, and a basal injection history of the subject. In some embodiments, subject parameters 512 comprises at least a body weight of the subject, a plurality of glucose measurements of the subject taken over the time course, an injection history of the subject, an overbasalisation limit of the subject, a last injection data refresh for the subject, an upper limit target glucose range of the subject, and a lower limit target glucose range of the subject.

Orchestrator 230, within the container engine 70, organizes the response to the medication dose guidance request 510 based on the subject parameters 512 the function requests 254 in the medication dose guidance request 510. Orchestrator 230 identifies which functions 608 are needed based on the subject parameters 512. The required functions are embedded in the selected containers 74 by an encoder module 240 (not shown). Orchestrator 203 further determines if there is an order in which the functions need to be performed. For example, container 74-4 with the data reconstruction embedded function 608-4 can be scheduled to execute function requests before or in parallel to container 74-2 with the adherence embedded function 608-2, as these functions can be executed independently of one another. However, in some embodiments, a container in the set of container 74-2, 74-3, and 74-4 may depended on container 74-1 with the calculation embedded function 608-1 (e.g. the adherence embedded function 608-2 in container 74-2 may require some calculations, which may, in some embodiments, be performed by the calculation embedded function 608-1 in container 74-1). Orchestrator 230 may trigger the addition of computational resources (e.g. additional containers 74 in any particular container class) on an as needed basis. For example, container 74-4 with the embedded function for data reconstruction 608-4 can have additional resources (e.g. more containers 74 in the same container class) dedicated to it thereby ensuring that the response to the medication dose guidance request 510 doesn't time out.

In some embodiments, when more computational resources are required for a function request, orchestrator 230 instantiates containers in the container class that can execute the respective function for that function request.

While orchestrator 230 oversees the progress of each container 74, the containers may exchange information between themselves to facilitate the execution of their respective function requests 254. Each container 74 in the plurality of containers communicates with other containers via an application programming interface (API) in accordance with a micro-service architecture at the calling of orchestrator 230. In some embodiments, the orchestrator 230 is any of a plurality of commercially available orchestrators, including but not limited to Azure Service Fabric, Kubernetes, Docker Swarm and Mesosphere DC/OS. In some embodiments, orchestrator 230 is a proprietary orchestrator that is not commercially available.

Each container 74 produces a function result 256 for the respective function request 254. The plurality of function results is used to provide a medication dose guidance recommendation 610. In some embodiments, another container (e.g. 74-5, not shown) may be used to compile and combine the plurality of function requests into a medication dose guidance recommendation 610 to the subject. In some embodiments, orchestrator 230 performs the compilation of the function results and provides the medication dose guidance recommendation 610 to the subject.

## REFERENCES CITED AND ALTERNATIVE EMBODIMENTS

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation of patent documents herein is done for convenience only and does not reflect any view of the validity, patentability, and/or enforceability of such patent documents.

The present invention can be implemented as a computer program product that comprises a computer program mechanism embedded in a non-transitory computer readable storage medium. For instance, the computer program product could contain the program modules shown in any combination of figs. 1 and 2 and/or described in fig. 8. These program modules can be stored on a CD-ROM, DVD, magnetic disk storage product, USB key, or any other non-transitory computer readable data or program storage product.

Many modifications and variations of this invention can be made without departing from its scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims.

## Claims

1. A computing system for providing to a plurality of subjects medication dosing guidance recommendations for a dose of insulin medicament to achieve a predetermined blood glucose target range of a subject to treat a diabetes mellitus condition using a multi-function dose guidance algorithm comprising a plurality of functions, wherein each respective function in the plurality of functions is associated with a corresponding container class, and wherein the computing system comprises one or more processors and a memory, the memory comprising:

   - instructions that, when executed by the one or more processors, perform a method responsive to receiving a medication dose guidance request from a subject in the plurality of subjects, the method comprising the steps of:

      (i) identifying a plurality of function requests required to satisfy the medication dose guidance request, wherein a function request in the plurality of function requests is executable by a corresponding function in the plurality of functions,
      (ii) using an encoder module to embed, for each respective function request in the plurality of function requests, the respective function request and associated input data into a container of the container class associated with the function corresponding to the respective function request,
      (iii) collecting, for each respective function request in the plurality of function requests, a function result from the container that executed the respective function request, thereby obtaining a plurality of function results, and
      (iv) providing a medication dose guidance recommendation using the plurality of function results,

   wherein:

      - a composite resource requirement for the containers of a given container class is evaluated on a recurring basis,
      - when the composite resource requirement for the containers of the given container class satisfies a resource allocation threshold associated with the given container class, one or more additional containers of the given container class is added and permitted to accept function requests from the encoder module that match the given container class,
      - when the composite resource requirement for the containers of the given container class satisfies a second resource allocation threshold associated with the given container class, one or more containers of the given container class is removed and no longer permitted to accept function requests from the encoder module,
      - the medication dose guidance request is for a dose of insulin medicament, and
      - the dose of an insulin medicament is to achieve a predetermined blood glucose target range of a subject to treat a diabetes mellitus condition.

2. The computing system as in claim 1, wherein:

      - the plurality of functions comprises a first function and a second function,
      - the first function is executable by each container of a first container class,
      - the second function is executable by each container of a second container class, and
      - a hardware resource requirement of the first function is different from a hardware resource requirement of the second function.

3. The computing system as in any claim 1 or 2, wherein:

- the plurality of functions comprises a first function and a second function,
- the first function is executable by each container of a first container class,
- the second function is executable by each container of a second container class,
- each container of the first container class is hosted by server in a first set of servers, and
- each container of the second container class is hosted by a server in second set of servers other than the first set of servers.

4. The computing system as in any of claims 1-3, wherein:

- a given container of a container class associated with the function corresponding to a given function request evaluates whether the respective function request contains input data required for the function to provide a function result, and
- when the function request fails to contain the input data required, no function result is provided by the given container of the container class associated with the function.

5. The computing system as in any of claims 1-4, wherein the system concurrently executes a medication dose guidance request for each subject in the plurality of subjects.

6. The computing system as in claim 5, wherein the plurality of subjects comprises at least 10,000 subjects.

7. The computing system as in any of claims 1-6, wherein the plurality of functions comprises one or more of:

- calculating a medication dose guidance recommendation of a subject,
- reconstructing an insulin injection history of a subject,
- calculating a titration glucose level of a subject,
- reconstructing a blood glucose history of a subject, and
- getting dose guidance parameters of a subject.

8. The computing system as in any of claims 1-7 wherein the method comprises the further step of:

- performing a quality check of the dose guidance request, wherein the quality check fails and results in no medication dose guidance recommendation being provided when:

(a) a dose guidance request from a subject comprises a record of a hypoglycemic event, or
(b) a dose guidance request from a subject comprises a record indicating that the subject has failed to take a previous recommended medication dose.

9. The computing system of claim 1, wherein the encoder module (ii) further comprises evaluating each respective function for a resource requirement.

10. The computing system as in any of claims 1-9, wherein at least two functions for a subject are concurrently executed.

11. The computing system as in any of claims 1-10, wherein at least two functions for a subject are executed sequentially.

12. The computing system as in any of claims 1-11, wherein the input required by the function request comprises, for a given subject, at least:

- an upper limit target glucose range of the subject,
- a blood glucose history of the subject,
- a basal injection history of the subject, and
- a most recent medication dose guidance recommendation,

and optionally one or more of:

- a body weight of the subject,
- a lower limit target glucose range of the subject,

- an overbasalisation limit of the subject, and
- a starting insulin basal dose of the subject.

**Patentansprüche**

1. Computersystem zum Bereitstellen von Medikamentendosis-Anleitungsempfehlungen für eine Insulinmedikamentendosis an eine Vielzahl von Personen, um einen vorbestimmten Blutglukose-Zielbereich einer Person zu erreichen, um einen Diabetes-mellitus-Zustand unter Verwendung eines multifunktionalen Dosisanleitungsalgorithmus zu behandeln, der eine Vielzahl von Funktionen umfasst, wobei jede jeweilige Funktion in der Vielzahl von Funktionen mit einer entsprechenden Behälterklasse assoziiert ist, und wobei das Computersystem einen oder mehrere Prozessoren und einen Speicher umfasst, der Speicher umfassend:

   - Anweisungen, die bei Ausführung durch den einen oder die mehreren Prozessoren ein Verfahren in Reaktion auf das Empfangen einer Medikamentendosis-Anleitungsanforderung von einer Person aus der Vielzahl von Personen durchführen, das Verfahren die folgenden Schritte umfassend:

      (i) Identifizieren einer Vielzahl von Funktionsanforderungen, die erforderlich sind, um die Medikamentendosis-Anleitungsanforderung zu erfüllen, wobei eine Funktionsanforderung in der Vielzahl von Funktionsanforderungen durch eine entsprechende Funktion in der Vielzahl von Funktionen ausführbar ist,
      (ii) Verwenden eines Codiermoduls, um, für jede jeweilige Funktionsanforderung in der Vielzahl von Funktionsanforderungen, die jeweilige Funktionsanforderung und die assoziierten Eingabedaten in einen Behälter der Behälterklasse, die mit der der jeweiligen Funktionsanforderung entsprechenden Funktion assoziiert ist, einzubetten,
      (iii) Sammeln, für jede jeweilige Funktionsanforderung in der Vielzahl von Funktionsanforderungen, eines Funktionsergebnisses von dem Behälter, der die jeweilige Funktionsanforderung ausführte, wodurch eine Vielzahl von Funktionsergebnissen erhalten wird, und
      (iv) Bereitstellen einer Medikamentendosis-Anleitungsempfehlung unter Verwendung der Vielzahl von Funktionsergebnissen,

   wobei:

      - ein zusammengesetzter Ressourcenbedarf für die Behälter einer gegebenen Behälterklasse auf wiederkehrender Basis beurteilt wird,
      - wenn das zusammengesetzte Ressourcenerfordernis für die Behälter der gegebenen Behälterklasse einen mit der gegebenen Behälterklasse assoziierten Ressourcenzuteilungsschwellenwert erfüllt, ein oder mehrere zusätzliche Behälter zu der gegebenen Behälterklasse hinzugefügt werden und es ihnen gestattet wird, Funktionsanforderungen von dem Codiermodul, die mit der gegebenen Behälterklasse übereinstimmen, anzunehmen,
      - wenn das zusammengesetzte Ressourcenerfordernis für die Behälter der gegebenen Behälterklasse einen mit der gegebenen Behälterklasse assoziierten zweiten Ressourcenzuteilungsschwellenwert erfüllt, ein oder mehrere zusätzliche Behälter aus der gegebenen Behälterklasse entfernt werden und es ihnen nicht mehr gestattet ist, Funktionsanforderungen von dem Codiermodul anzunehmen,
      - die Medikamentendosis-Anleitungsanforderung für eine Insulin-Medikamentendosis ist, und
      - die Dosis eines Insulinmedikaments dazu dient, einen vorbestimmten Blutglukose-Zielbereich einer Person zu erreichen, um einen Diabetes-mellitus-Zustand zu behandeln.

2. Computersystem nach Anspruch 1, wobei:

   - die Vielzahl von Funktionen eine erste Funktion und eine zweite Funktion umfasst,
   - die erste Funktion von jedem Behälter einer ersten Behälterklasse ausführbar ist,
   - die zweite Funktion von jedem Behälter einer zweiten Behälterklasse ausführbar ist, und
   - sich ein Hardwareressourcenerfordernis der ersten Funktion von einem Hardwareressourcenerfordernis der zweiten Funktion unterscheidet.

3. Computersystem nach Anspruch 1 oder 2, wobei:

   - die Vielzahl von Funktionen eine erste Funktion und eine zweite Funktion umfasst,

- die erste Funktion von jedem Behälter einer ersten Behälterklasse ausführbar ist,
- die zweite Funktion von jedem Behälter einer zweiten Behälterklasse ausführbar ist,
- jeder Behälter der ersten Behälterklasse von einem Server in einer ersten Gruppe von Servern gehostet wird, und
- jeder Behälter der zweiten Behälterklasse von einem Server in einer zweiten Gruppe von Servern, die sich von der ersten Gruppe von Servern unterscheidet, gehostet wird.

4. Computersystem nach einem der Ansprüche 1-3, wobei:

- ein gegebener Behälter einer mit der Funktion assoziierten Behälterklasse, die einer gegebenen Funktionsanforderung entspricht, bewertet, ob die jeweilige Funktionsanforderung Eingabedaten enthält, die erforderlich sind, damit die Funktion ein Funktionsergebnis bereitstellt, und
- wenn die Funktionsanforderung die erforderlichen Eingabedaten nicht enthält, kein Funktionsergebnis von dem jeweiligen Behälter der mit der Funktion assoziierten Behälterklasse bereitgestellt wird.

5. Computersystem nach einem der Ansprüche 1-4, wobei das System gleichzeitig eine Medikamentendosis-Anleitungsanforderung für jede Person der Vielzahl von Personen ausführt.

6. Computersystem nach Anspruch 5, wobei die Vielzahl von Personen mindestens 10.000 Personen umfasst.

7. Computersystem nach einem der Ansprüche 1-6, wobei die Vielzahl von Funktionen eines oder mehrere der Folgenden umfasst:

- Berechnen einer Medikamentendosis-Anleitungsempfehlung einer Person,
- Rekonstruieren einer Insulininjektionshistorie einer Person,
- Berechnen eines Titrationsglukosespiegels einer Person,
- Rekonstruieren einer Blutglukosehistorie einer Person, und
- Erhalten von Dosisanleitungsparametern einer Person.

8. Computersystem nach einem der Ansprüche 1-7, wobei das Verfahren folgenden weiteren Schritt umfasst:

- Durchführen einer Qualitätsüberprüfung der Dosisanleitungsanforderung, wobei die Qualitätsüberprüfung fehlschlägt und bewirkt, dass keine Medikamentendosis-Anleitungsempfehlung bereitgestellt wird, wenn:

(a) eine Dosisanleitungsanforderung von einer Person eine Aufzeichnung eines hypoglykämischen Ereignisses umfasst, oder
(b) eine Dosisanleitungsanforderung von einer Person eine Aufzeichnung umfasst, die angibt, dass die Person eine vorherige empfohlene Medikamentendosis nicht eingenommen hat.

9. Computersystem nach Anspruch 1, wobei das Codiermodul (ii) ferner das Bewerten jeder jeweiligen Funktion für ein Ressourcenerfordernis umfasst.

10. Computersystem nach einem der Ansprüche 1-9, wobei mindestens zwei Funktionen für eine Person gleichzeitig ausgeführt werden.

11. Computersystem nach einem der Ansprüche 1-10, wobei mindestens zwei Funktionen für eine Person sequentiell ausgeführt werden.

12. Computersystem nach einem der Ansprüche 1-11, wobei die von der Funktionsanforderung angeforderte Eingabe für eine gegebene Person mindestens Folgendes umfasst:

- einen oberen Grenzwert für den Zielglukosebereich der Person,
- eine Blutglukosehistorie der Person,
- eine Basalinjektionshistorie der Person, und
- eine jüngste Medikamentendosisanleitung,

und optional eines oder mehrere von:

- einem Körpergewicht der Person,
- einem unteren Grenzwert für den Glukosezielbereich der Person,
- einem Überbasalisierungsgrenzwert der Person, und
- einer Ausgangsinsulinbasaldosis der Person.

**Revendications**

1. Système informatique destiné à fournir à une pluralité de recommandations d'indication posologique de médicament de sujets pour une dose de médicament insulinique afin d'obtenir une plage cible de glycémie prédéterminée d'un sujet pour traiter une affection de diabète sucré à l'aide d'un algorithme d'indication de dose multifonction comprenant une pluralité de fonctions, dans lequel chaque fonction respective de la pluralité de fonctions est associée à une classe de conteneurs correspondante, et dans lequel le système informatique comprend un ou plusieurs processeurs et une mémoire, la mémoire comprenant :

   - des instructions qui, lorsqu'elles sont exécutées par les un ou plusieurs processeurs, réalisent un procédé en réponse à la réception d'une demande d'indication de dose de médicament en provenance d'un sujet dans la pluralité de sujets, le procédé comprenant les étapes consistant à :

      (i) identifier une pluralité de demandes de fonction requises pour satisfaire la demande d'indication de dose de médicament, dans lequel une demande de fonction dans la pluralité de demandes de fonction est exécutable par une fonction correspondante dans la pluralité de fonctions,
      (ii) utiliser un module codeur pour intégrer, pour chaque demande de fonction respective dans la pluralité de demandes de fonction, la demande de fonction respective et des données d'entrée associées dans un conteneur de la classe de conteneurs associée à la fonction correspondant à la demande de fonction respective,
      (iii) collecter, pour chaque demande de fonction respective dans la pluralité de demandes de fonction, un résultat de fonction à partir du conteneur qui a exécuté la demande de fonction respective, pour ainsi obtenir une pluralité de résultats de fonction, et
      (iv) fournir une recommandation d'indication de dose de médicament à l'aide de la pluralité de résultats de fonction,

   dans lequel :

      - une exigence de ressources composites pour les conteneurs d'une classe de conteneurs donnée est évaluée de manière récurrente,
      - lorsque l'exigence de ressources composites pour les conteneurs de la classe de conteneurs donnée satisfait un seuil d'allocation de ressources associé à la classe de conteneurs donnée, un ou plusieurs conteneurs supplémentaires de la classe de conteneurs donnée sont ajoutés et autorisés à accepter des demandes de fonction en provenance du module codeur qui concordent avec la classe de conteneurs donnée,
      - lorsque l'exigence de ressources composites pour les conteneurs de la classe de conteneurs donnée satisfait un deuxième seuil d'allocation de ressources associé à la classe de conteneurs donnée, un ou plusieurs conteneurs de la classe de conteneurs donnée sont retirés et ne sont plus autorisés à accepter des demandes de fonction provenant du module codeur,
      - la demande d'indication de dose de médicament concerne une dose de médicament insulinique, et
      - la dose d'un médicament insulinique doit atteindre une plage cible de glycémie prédéterminée d'un sujet pour traiter une affection de diabète sucré.

2. Système informatique selon la revendication 1, dans lequel :

   - la pluralité de fonctions comprend une première fonction et une deuxième fonction,
   - la première fonction est exécutable par chaque conteneur d'une première classe de conteneurs,
   - la deuxième fonction est exécutable par chaque conteneur d'une deuxième classe de conteneurs, et
   - une exigence de ressources matérielles de la première fonction est différente d'une exigence de ressources matérielles de la deuxième fonction.

3. Système informatique selon l'une quelconque des revendications 1 ou 2, dans lequel :

- la pluralité de fonctions comprend une première fonction et une deuxième fonction,
- la première fonction est exécutable par chaque conteneur d'une première classe de conteneurs,
- la deuxième fonction est exécutable par chaque conteneur d'une deuxième classe de conteneurs,
- chaque conteneur de la première classe de conteneurs est hébergé par un serveur dans un premier ensemble de serveurs, et
- chaque conteneur de la deuxième classe de conteneurs est hébergé par un serveur dans un deuxième ensemble de serveurs autre que le premier ensemble de serveurs.

4. Système informatique selon l'une quelconque des revendications 1 à 3, dans lequel :

- un conteneur donné d'une classe de conteneurs associée à la fonction correspondant à une demande de fonction donnée évalue si la demande de fonction respective contient des données d'entrée requises pour que la fonction fournisse un résultat de fonction, et
- lorsque la demande de fonction ne contient pas les données d'entrée requises, aucun résultat de fonction n'est fourni par le conteneur donné de la classe de conteneurs associée à la fonction.

5. Système informatique selon l'une quelconque des revendications 1 à 4, dans lequel le système exécute simultanément une demande d'indication de dose de médicament pour chaque sujet dans la pluralité de sujets.

6. Système informatique selon la revendication 5, dans lequel la pluralité de sujets comprend au moins 10 000 sujets.

7. Système informatique selon l'une quelconque des revendications 1 à 6, dans lequel la pluralité de fonctions comprend un ou plusieurs parmi :

- le calcul d'une recommandation d'indication de dose de médicament d'un sujet,
- la reconstruction d'un historique d'injection d'insuline d'un sujet,
- le calcul du taux de glucose titré chez un sujet,
- la reconstruction d'un historique de glycémie d'un sujet, et
- l'obtention de paramètres d'indication de dose d'un sujet.

8. Système informatique selon l'une quelconque des revendications 1 à 7, dans lequel le procédé comprend l'étape supplémentaire consistant à :

- réaliser un contrôle de qualité de la demande d'indication de dose, dans lequel le contrôle de qualité échoue et n'entraîne la fourniture d'aucune recommandation d'indication de dose de médicament lorsque :

(a) une demande d'indication de dose en provenance d'un sujet comprend un enregistrement d'un événement hypoglycémique, ou
(b) une demande d'indication de dose en provenance d'un sujet comprend un enregistrement indiquant que le sujet n'a pas pris une dose de médicament recommandée précédente.

9. Système informatique selon la revendication 1, dans lequel le module codeur (ii) comprend en outre l'évaluation de chaque fonction respective pour une exigence de ressources.

10. Système informatique selon l'une quelconque des revendications 1 à 9, dans lequel au moins deux fonctions pour un sujet sont exécutées simultanément.

11. Système informatique selon l'une quelconque des revendications 1 à 10, dans lequel au moins deux fonctions pour un sujet sont exécutées séquentiellement.

12. Système informatique selon l'une quelconque des revendications 1 à 11, dans lequel l'entrée requise par la demande de fonction comprend, pour un sujet donné, au moins :

- une plage de glucose cible limite supérieure du sujet,
- un historique de glycémie du sujet,
- un historique d'injection basale du sujet, et
- une recommandation d'indication de dose de médicament la plus récente,

**EP 3 844 765 B1**

et éventuellement un ou plusieurs parmi :

- un poids corporel du sujet,
- une plage de glucose cible limite inférieure du sujet,
- une limite de surdosage d'injection basale du sujet, et
- une dose d'insuline basale de départ du sujet.

# Fig. 1A

```
┌─────────────────┐                    ╱───╲
│      Input      │                   │  A  │
└─────────────────┘                    ╲───╱
         │                               ▲
         ▼                               │
┌─────────────────┐              ┌─────────────────┐
│       1.1       │              │       1.8       │
└─────────────────┘              └─────────────────┘
         │                               ▲
         ▼                               │
┌─────────────────┐              ┌─────────────────┐
│       1.2       │              │       1.7       │
└─────────────────┘              └─────────────────┘
         │                               ▲
         ▼                               │
┌─────────────────┐              ┌─────────────────┐
│       1.3       │              │       1.6       │
└─────────────────┘              └─────────────────┘
         │                               ▲
         ▼                               │
┌─────────────────┐              ┌─────────────────┐
│       1.4       │─────────────▶│       1.5       │
└─────────────────┘              └─────────────────┘
```

# Fig. 1B

# Fig. 2A

# Fig. 2B

# Fig. 3

# Fig. 4A

Container Engine 70-1

Orchestrator 230-1 | Container 74-1-1 | Container 74-1-2 | ... | Container 74-1-Z

Container Engine 70-E

Orchestrator 230-E | Container 74-E-1 | Container 74-E-2 | ... | Container 74-E-Z

602

Data transmission device 150

48

Communication Network(s) 106

110-1

Subject user device 1

...

110-Q

Subject user device Q

## Fig. 4B

Medication dose guidance computer system 602

48

502

Data transmission device 150

Communication Network(s)
106

102-1

Glucose sensor 1

⋮

102-P

Glucose sensor P

104-1

Insulin pen 1

⋮

104-Q

Insulin pen Q

\*

# Fig. 5

# Fig. 6

(302) A computing system for providing medication dosing guidance recommendations to a plurality of subjects using a multi-function dose guidance algorithm comprising a plurality of functions, wherein each respective function in the plurality of functions is associated with a container class in a plurality container classes, and wherein the computing system comprises one or more processors and a memory, the memory comprising: instructions that, when executed by the one or more processors, perform a method responsive to receiving a medication dose guidance request from a subject in the plurality of subjects

> (304) The plurality of functions comprises a first function and a second function
>
> > (306) The first function is executable by each instance of a container of a first container class in the plurality of container classes
> >
> > (308) The second function is executable by each instance of a container of a second container class in the plurality of container classes
> >
> > (310) A hardware resource requirement of the first function is different than a hardware resource requirement of the second function

(312) Identify a plurality of function requests required to satisfy the medication dose guidance request, wherein function requests in the plurality of function requests is executable by a corresponding function in the plurality of functions

(314) Use an encoder module to embed, for each respective function request in the plurality of function requests, the respective function request into a container in a container class associated with the function corresponding to the respective function request

(316) Collect, for each respective function request in the plurality of function requests, a function result from the container in the container class that executed the respective function request, thereby obtaining a plurality of functions results

(318) Provide a medication dose guidance recommendation using the plurality of functions results

\*

# Fig. 7

400

```
                    ┌───────────┐
                    │No Guidance│
                    │    405    │
                    └─────▲─────┘
                          │
                          No
                          │
┌──────────┐  ┌──────────┐      ┌──────────┐  ┌──────────────┐  ┌──────────┐  ┌──────────┐  ┌────────────┐
│   Dose   │  │  Valid   │      │ Identify │  │  Determine   │  │  Embed   │  │  Assign  │  │Each function│
│ guidance │  │ Request  │─Yes─▶│ Function │─▶│  Resource    │─▶│ Required │─▶│ Function │─▶│  request   │
│ request  │─▶│  Check   │      │ Requests │  │Requirement   │  │Functions in│ │Requests to│ │  contains  │
│ received │  │   404    │      │   406    │  │  for each    │  │Containers │  │Containers │  │  required  │
│   402    │  │          │      │          │  │  Function    │  │   410    │  │   412    │  │  dataset   │
└──────────┘  └──────────┘      └──────────┘  │   Request    │  └──────────┘  └──────────┘  │   407      │
                                              │    408       │                              └────────────┘
                                              └──────────────┘
```

No Guidance 405

No

Yes

```
┌──────────┐  ┌──────────┐      ┌──────────────┐      ┌──────────┐
│ Provide  │  │Containers│      │     More     │      │Containers│
│medication│  │  return  │      │ computational│      │ execute  │
│   dose   │◀─│ function │◀─No──│resources needed│◀────│ function │
│ guidance │  │ results  │      │for any function│     │ requests │
│   418    │  │   416    │      │   requests?  │      │   414    │
└──────────┘  └──────────┘      │     409      │      └──────────┘
                                └──────────────┘           ▲
                                   Yes                     │
                                    │  ┌──────────────┐    │
                                    └─▶│Allocate more │────┘
                                       │containers for said│
                                       │function request│
                                       │     411      │
                                       └──────────────┘
```

EP 3 844 765 B1

## Fig. 8

# Fig. 9

Medication dose guidance request 510

602

Medication dose guidance recommendation 610

Container Engine 70

Orchestrator 230-1

Container 74-1

Container 74-2

Container 74-3

Container 74-4

Subject Parameters 512-1

Embedded Function - Calculation 608-1

Embedded Function - Adherence 608-2

Embedded Function - Titration Glucose Level 608-3

Embedded Function - Data Reconstruction 608-4

Function Requests 254

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170091419 A
- US 2018165129 A
- WO 2018064568 A
- US 2017302589 A
- US 20190035500 A
- WO 2018228932 A
- EP 18198410
- US 20170063833 A
- US 20180014776 A1
- EP 2017065578 W
- US 20040153257 A1

**Non-patent literature cited in the description**

- **BERGENSTAL et al.** Can a Tool that Automates Insulin Titration be a Key to Diabetes Management?. *Diabetes Tech. and Thera.,* 2012, vol. 14 (8), 675-682
- **HOLMAN et al.** , "10-year follow-up of intensive glucose control in type 2 diabetes. *N. Engl. J. Med.,* 2008, vol. 359, 1577-1589
- **KHUNTI et al.** Self-titration of insulin in the management of people with type 2 diabetes: a practical solution to improve management in primary care. *Diabetes, Obes., and Metabol.,* 2012, vol. 15 (8), 690-700
- **NORRIS et al.** , "Self-management education for adults with type 2 diabetes: a meta-analysis on the effect of glycemic control. *Diabetes Care,* 2002, vol. 25, 1159-71
- **KULZER et al.** Effects of self-management training in type 2 diabetes: a randomized, prospective trial. *Diabet. Med.,* 2007, vol. 24, 415-23
- **ANDERSON et al.** , "Patient empowerment: results of a randomized controlled trial. *Diabetes Care.,* 1995, vol. 18, 943-9
- **EDELMAN et al.** , *"AUTONOMY: The First Randomized Trial Comparing Two Patient-Driven Approaches to Initiate and Titrate Prandial Insulin Lispro in Type 2 Diabetes,* 2014, vol. 37 (8), 2132-2140
- **ARNOLDS et al.** , "Common Standards of Basal Insulin Titration in T2DM. *J. Diabetes Sci. Technol.,* 2013, vol. 7 (3), 771-788
- **BERRIMAN et al.** The Application of Cloud Computing to Scientific Workflows: A Study of Cost and Performance. *Phil. Trans. R. Soc. A,* 2013, vol. 371, 20120066
- **LIM et al.** Automated Control in Cloud Computing: Challenges and Opportunities. *ACD'09, Barcelona,* 2009
- **K.-D. K. B. T. GOUGH DA.** Frequency characterization of blood glucose dynamics. *Ann Biomed Eng.,* 2003, vol. 31, 91-97
- **HOGG.** Software Containers: Used More Frequently than Most Realize. *Network World,* 26 May 2016
- **SCHMID.** New options in insulin therapy. *J Pediatria (Rio J).,* 2007, vol. 83 (5), S146-S155
- **DUNN et al.** An Updated Review of its Use in the Management of Diabetes Mellitus. *Drugs,* 2003, vol. 63, 1743
- **PLANK et al.** A double-blind, randomized, dose-response study investigating the pharmacodynamic and pharmacokinetic properties of the long-acting insulin analog detemir. *Diabetes Care,* 2005, vol. 28, 1107-1112